# EUROPEAN PATENT APPLICATION

(11) **EP 3 819 377 A1**
(43) Date of publication of application: **12.05.2021**
(21) Application number: 19208168.5
(22) Date of filing: 08.11.2019
(51) Int. Cl.: C12N 15/113, C12N 15/11

(54) **CIRCULAR RNA AND USES THEREOF FOR INHIBITING RNA-BINDING PROTEINS**

(71) Applicant: Justus-Liebig-Universität Gießen, 35390 Gießen (DE)
(72) Inventor: BINDEREIF, Albrecht, 35394 Gießen (DE); SCHREINER, Silke, 35469 Allendorf/Lumda (DE)
(74) Representative: Stumpf, Peter

(57) **Abstract**

The present invention relates compositions and methods for capturing RNA-binding proteins using a circular RNA molecule, preferably in a sequence-specific manner, such that the functional activity of the RNA-binding proteins is inhibited.

## Description

### INTRODUCTION

The present invention relates compositions and methods for capturing RNA-binding proteins using a circular RNA (circRNA) molecule, preferably in a sequence-specific manner, such that the functional activity of the RNA-binding proteins is inhibited in their respective metabolic processes.

### BACKGROUND OF THE INVENTION

Circular RNAs (circRNAs) are a novel class of noncoding RNAs, which are generated from pre-mRNAs by circular splicing (also termed "back splicing") of one or several adjacent exons (Wilusz et al., 2018, Wiley Interdiscip Rev RNA 9(4):e1478). CircRNAs are expressed in a cell-type specific manner, are much more stable than the corresponding linear mRNA, evolutionarily conserved, and are mostly cytoplasmic. Although present in all eukaryotes investigated so far, circRNAs are still largely undefined in functional terms. The fact that circRNAs mainly are derived from conserved exonic sequences raised the general question whether they might have protein-coding potential, as a few recent reports suggest (Legnini et al., 2017, Mol Cell 66(1):22-37; Pamudurti et al., 2017, Mol Cell 66(1):9-21; Yang et al., 2017, Cell Res 7(5):626-641). However, what the biological significance of circRNA translation might be, and how widespread its occurrence, is currently still a matter of dispute (Schneider and Bindereif, 2017, Cell Res 27(6):724-725). Several other, hypothetical roles have been discussed for circRNAs, for example allostery, complex assembly, antisense activity, and protein sponging (Hentze and Preiss, 2013, EMBO J 32(7):923-925). However, no natural examples for these functions are known so far. The high stability of natural circRNAs has recently provoked studies of using circRNAs as a basis for constructing designer artificial circRNAs for novel therapeutic applications. In particular, circRNAs were successfully developed as miRNA sponges to interfere with specific gene expression (for example, see Jost et al., 2018, RNA Biol 15(8): 1032-1039).

RNA-binding proteins (RBPs) are proteins that bind to double- or single-stranded RNA in cells and participate in forming ribonucleoprotein complexes. RBPs are found in the cytoplasm as well as in the nucleus and have crucial roles in various cellular processes such as cellular function, transport and localization of RNA. RBPs play an especially major role in post-transcriptional control of RNAs, such as splicing, polyadenylation, mRNA stabilization, mRNA localization and translation.

Eukaryotic cells encode for approximately 500 genes with unique RNA-binding activity and protein-protein interactions. As RNA emerges from RNA polymerase, RNA transcripts are immediately covered with RNA-binding proteins that regulate every aspect of RNA metabolism and function including RNA biogenesis, maturation, transport, cellular localization and stability. All RBPs bind RNA; however, they do so with different sequence specificities and affinities, which allows the RBPs to be as diverse as their targets and functions (Glisovic et al., 2008, FEBS Letters 582(14):1977-1986). These targets include protein-encoding mRNA, as well as a number of non-coding RNAs (ncRNAs), which almost always function as ribonucleoprotein complexes and not as naked RNAs. These ncRNAs include microRNAs, small interfering RNAs (siRNA), as well as spliceosomal small nuclear RNAs (snRNA) (Matera et al., 2007, Nat Rev Molecular Cell Biology 8(3): 209-220).

RBPs are emerging to play a crucial role in tumor development and many RBPs are differentially expressed in different cancer types, for example, KHDRBS1 (Sam68), ELAVL1 (HuR) and FXR1 (Bielli et al., 2011, Endocrine-Related Cancer 18(4):R91-R102; Liao et al., 2013, BMC Gastroenterology 13:126; Frisone et al., 2015, BioMed Research International 2015:528954; Abdelmohsen et al., 2010, Wiley Interdisciplinary Reviews: RNA 1(2):214-229; Wang et al., 2013, International Journal of Molecular Sciences 14(5):10015-10041; and Qian et al., 2015, Proc. Nat. Acad. Sci. USA 112(11):3469-3474). For some RBPs, the change in expression is related to copy number variations (CNV), for example CNV gains of BYSL in colorectal cancer cells, ESRP1 and CELF3 in breast cancer, RBM24 in liver cancer, and IGF2BP2 and IGF2BP3 in lung cancer (Wang et al., 2018, Cell Reports 22(1):286-298; Sebestyén et al., 2016, Genome Research 26(6):732-744).

Heterogeneous nuclear ribonucleoprotein (hnRNP) L represents a classical RNA-binding protein with four RNA-recognition motifs (RRMs) and well-documented RNA-binding specificity and function in splicing regulation (Piñol-Roma et al., 1989, J Cell Biol 109(6 Pt 1):2575-2587; Hui et al., 2003, Nat Struct Biol 10(1):33-37; Hui et al., 2005, EMBO J 24(11):1988-1998, Hung et al., 2008, RNA 14(2):284-96). Specifically, hnRNP L can act either as splice activator or repressor, requiring binding to short CA-repeat or CA-rich RNA elements, which often reside in intronic positions near splice sites. Further, hnRNP L has been shown to be overexpressed in cancers, such as pancreatic cancer.

RNA-binding proteins are becoming targets for therapeutic applications in which their inhibition results in treatment of diseases where the RNA-binding proteins are overexpressed or where their specific activity is desired to be inhibited, such as in cancer. There remains a need for new approaches for targeting RNA-binding proteins involved in cell growth processes, such that the functional activity of these RNA-binding proteins can be inhibited, which in turn prevents cell growth. This need, however, is met by the present invention.

### SUMMARY OF THE INVENTION

The present invention relates to a circular RNA molecule to which one or more copies of an RNA-binding protein binds, thereby allowing for the capture/sequestering of the RNA-binding protein in order to inhibit its function. The present invention is based, at least in part, on the inventors' novel and unexpected finding that circular RNA molecules (circRNAs) can act as protein sponges, *i.e.,* that they can specifically bind and functionally inactivate RNA-binding proteins. As described herein, the inventors have shown that heterogeneous nuclear ribonucleoprotein L (hnRNP L), an exemplary RNA-binding protein involved in regulating splicing, binds to a circular RNA molecule in a sequence specific manner, and, due to this binding, the function/activity of hnRNP L as a splicing regulator is inhibited. In view of this inhibition, splicing networks can be efficiently modulated, in particular with regard to target genes that are subject to alternative splicing, such that new therapeutic strategies in molecular medicine are now available.

In one embodiment, the present invention is directed to a circular RNA molecule, which comprises a binding domain having a nucleotide sequence to which one or more copies of an RNA-binding protein binds. Preferably, the binding results in inhibiting the function/activity of the RNA-binding protein. To be clear, the circular RNA itself does not inhibit the binding function of the RNA-binding protein to its normal binding sequence, but, rather, results in sequestering or capturing the RNA-binding protein such that it cannot perform its activity in the cellular process in which it normally functions. Therefore, the circular RNA molecules described herein are particularly suited for use in treating or preventing diseases or disorders where inhibition of the activity of an RNA-binding protein is desired. For example, since RNA-binding proteins are involved in cellular processes necessary for the growth of the cell, RNA-binding proteins can be targeted by a circular RNA of the invention in order to inhibit the activity of the RNA-binding protein and, thus, inhibit cell growth. In this way, diseases and disorders characterized by unwanted cell growth, such as cancer, can be treated or prevented. This is particular pertinent in situations where overexpression (or increased activity) of an RNA-binding protein is indicative of a disease or disorder, such as cancer. Thus, a circular RNA to which this overexpressed RNA-binding protein binds can be used in methods to treat or prevent the disease or disorder by capturing and inhibiting the activity of this RNA-binding protein.

An RNA-binding protein is a protein that binds to an RNA molecule, usually in a sequence-specific manner. The RNA to which an RNA-binding protein binds can be any form of RNA, whether single or double-stranded, coding or non-coding, including ribosomal RNA, messenger RNA, micro RNA, long non-coding RNA, enhancer RNA, small interfering RNA, signal recognition particle RNA, or intermediate or immature forms thereof. Such RNA-binding proteins are involved in many cellular processes, including transcription, splicing, RNA editing, translation, (viral) replication, mRNA localization, such that their inhibition will inhibit these processes. Exemplary RNA-binding proteins described herein include heterogenous nuclear ribonucleoprotein L (hnRNP L), cold inducible RNA-binding protein (CIRBP), STAU1, STAU2, Sam68, FXR1, BYSL, ESRP1, CELF3, RBM24, IGF2BP2, IGF2BP3, NSUN6, ZC3H13, ELAC1, RBMS3, ZGPAT, SF3B1, SRSF2, RBM10, U2AF1, SF3B1, PPRC1, RBMXL1, HNRNPCL1, and other hnRNP proteins (for a comprehensive list of RNA-binding proteins with known RNA-binding specificities, see Ray et al., 2013, Nature 499(7457): 172-177.

In one embodiment, the RNA-binding protein is heterogeneous nuclear ribonucleoprotein L. In one embodiment, the nucleotide sequence of the binding domain comprises CA or AC dinucleotides. Preferably, the number of CA or AC dinucleotides is at least 20, at least 30, at least 40, at least 50, at least 60, at least 75, at least 100, at least 125, at least 150, at least 175, at least 200, at least 250, at least 300, at least 350, at least 400, or at least 450 CA or AC dinucleotides. In a specific embodiment, the number of CA or AC dinucleotides is at least 90 CA or AC dinucleotides. Preferably, at least 4 of the CA or AC dinucleotides are consecutive.

In one embodiment, the total nucleotide sequence of the circular RNA is less than 2000 nucleotides, less than 1500 nucleotides, less than 1250 nucleotides, less than 1000 nucleotides, less than 500 nucleotides, or less than 250 nucleotides in length. In a specific embodiment, the circular RNA molecule comprises the nucleotide sequence (with the t residues replaced with u residues) (SEQ ID NO:1):

cacacacacacacacacacacacacacacacacacacacacacacacacacacacacacacacacacagatccacac

gcacacacacacacacacacacacacacacacacacacacacacacacacacacacacacacacacagatccacacg

acacacacagatccacacacacacacacacacacacacacacacacacacacacacacacacacacacacacac

acaca.

In a specific embodiment, the circular RNA molecule comprises the nucleotide sequence (with the T/t residues replaced with U/u residues) (SEQ ID NO:2):

AACCATGCCGAGTGCGGCCGCGGATCCatacatgacacacacacgcaatatatacatg
acacacacacgcaatatatacatgacacacacacgcaatatatacatgacacacacacgcaAGATGTGG
CCGCGGTCGGCGTGGACTGTAG

In a specific embodiment, the circular RNA molecule comprises the nucleotide sequence (with the T/t residues replaced with U/u residues) (SEQ ID NO:3):

AAAATCCGTTGACCTTAAACGGTCGTGTGGGTTCAAGTCCCTCCACC
CCCACGCCGGAAACGCAATAGCCGAAAAACAAAAAACAAAAAAAG
AGGATCcacacacacacacacacacacacacacacacacacacacacacacacacaca
gatccacacgcacacacacacacacacacacacacacacacacacacacacacacagatc
cacacacgacacacacagatccacacacacacacacacacacacacacacacacacacaca
cacacacacacacaAGATCCACTAGTAACGGCTCGAGAAAAACAAAAAA
CAAAACGGCTATTATGCGTTACCGGCGAGACGCTACGGACTT

In a specific embodiment, the circular RNA molecule comprises the nucleotide sequence (with the T/t residues replaced with U/u residues) (SEQ ID NO:4):

GGGAGACCCAAGCTTCCGTCGATTGTCCACTGGTCAACAATAGATG
ACTTACAACTAATCGGAAGGTGCAGAGACTCGACGGAGCTACCCT
AACGTCAAGACGAGGGTAAAGAGAGAGTCCAATTCTCAAAGCCAA
TAGGCAGTAGCGAAAGCTGCAAGAGAATGAAAATCCGTTGACCTTA
AACGGTCGTGTGGGTTCAAGTCCCTCCACCCCCACGCCGGAAACGC
AATAGCCGAAAAACAAAAAACAAAAAAAGAGGATCcacacacacacacaca
cacacacacacacacacacacacacacacacacacacacacacagatccacacgcacacacacacacac
acacacacacacacacacacacacacacacacacacacacacacagatccacacacgacacacacagatccaca

cacacacacacacacacacacacacacacacacacacacacacacacacacacacacacaAGATCCA
CTAGTAACGGCTCGAGAAAAACAAAAACAAAACGGCTATTATG
CGTTACCGGCGAGACGCTACGGACTTAAATAATTGAGCCTTAAAGA
AGAAATTCTTTAAGTGGATGCTCTCAAACTCAGGGAAACCTAAATC
TAGTTATAGACAAGGCAATCCTGAGCCAAGCCGAAGTAGTAATTAG
TAAGACCAGTGGACAATCGACGGATAACAGCATATCTAGA

In a specific embodiment, the circular RNA molecule comprises the nucleotide sequence (SEQ ID NO:5):
GGGAGUAAGC-(CA)₁₀-GCUUACAGUA.

In a specific embodiment, the circular RNA molecule comprises the nucleotide sequence (SEQ ID NO:6):
GGGAGUAAGC-(CA)₁₅-GCUUACAGUA.

In a specific embodiment, the circular RNA molecule comprises the nucleotide sequence (SEQ ID NO:7):
GGGAGUAAGC-(CA)₂₀-GCUUACAGUA.

In a specific embodiment, the circular RNA molecule comprises the nucleotide sequence (SEQ ID NO:8):
GGGAGUAAGCAUACAUGACACACACACGCAGCUUACAGUA.

In one embodiment, the circular RNA can be chemically modified to provide stability and/or to minimize any immune reaction caused by administration of the circular RNA to a patient. In one embodiment, the chemical modification is that the circular RNA comprises a modified ribonucleotide, for example, pseudouridine. In one embodiment, the chemical modification is that the circular RNA comprises a modified backbone, for example, the modified backbone comprises a phosphorothioate internucleotide linkage. In one embodiment, the circular RNA can be modified by comprising a modified ribonucleotide and a modified backbone.

In one embodiment, the circular RNA is purified. In one embodiment, the circular RNA is a recombinant/artificial construct. In one embodiment, the circular RNA is comprised within a pharmaceutical composition with a pharmaceutically acceptable carrier. Preferably, the circular RNA does not encode a peptide/protein/polypeptide (all used interchangeably) nor is a peptide/protein/polypeptide able to be expressed from the circular RNA.

The circular RNA molecule can be made by any appropriate method known in the art. For example, the RNA molecule can be transcribed *in vitro* from a DNA template, and the 5' and 3' ends of the RNA molecule can be ligated to form the circular RNA. Also, the circular RNA can be produced by cells in cell culture using the TORNADO system of Litke and Jaffrey (Litke and Jaffrey, 2019, Nature Biotechnology 37(6):667-675). The TORNADO system generates RNA transcripts that are efficiently processed into circular RNAs, which allows for micromolar concentrations in mammalian cells. Briefly, the TORNADO expression cassette encodes all of the required components for maturation of circular RNA in cells. In one particular example, transcription is driven by the highly active U6+27 promoter (which works in mammalian cells), and transcription termination is induced by the U-rich U6 terminator. Autocatalytic processing of the RNA transcript begins with cleavage of the two ribozymes that flank the RNA sequence of interest. Following cleavage of both ribozymes, the 5' and 3' ends of the RNA of interest contain unique 5' and 3' terminal ends. The nucleotides at the 5' and 3' ends hybridize to form a stem. This proximity helps to enhance the ligation efficiency mediated by the intracellular ligase, RtcB. After ligation, the RNA is circular and contains the RNA sequence of interest. Both of the above-described methods are used in the experimental results described herein.

In one embodiment, the present invention is directed to a method of treating or preventing a disease or disorder characterized by an increased amount or increased activity of an RNA-binding protein, comprising administering to a patient in need thereof a circular RNA comprising a binding domain having a nucleotide sequence to which one or more copies of the RNA-binding protein binds. In one embodiment, the present invention is directed to a method of treating or preventing a disease or disorder characterized by abnormal or unwanted cell growth, comprising administering to a patient in need thereof a circular RNA comprising a binding domain having a nucleotide sequence to which one or more copies of an RNA-binding protein binds, which RNA-binding protein is involved in or whose activity is necessary for cell growth. Optionally, a pharmaceutical composition comprising the circular RNA can be administered.

In one embodiment, the disease or disorder is cancer. The cancer can be characterized by one or more solid tumors. In one embodiment, the cancer is pancreatic cancer or oral squamous cell carcinoma.

In one embodiment, the circular RNA or pharmaceutical composition comprising the circular RNA can be administered naked or complexed with a carrier and can be administered by any suitable route, including intravenous or intraarterial administration or can be administered by injection into the solid tumor.

In one embodiment, the circular RNA molecule, optionally comprised within a pharmaceutical composition is for use as a medicament. In one embodiment, the use as a medicament can be for treating or preventing a disease or disorder characterized by an increased amount or increased activity of an RNA-binding protein.

In one embodiment, the present invention is directed to a method for capturing an RNA-binding protein, which method comprises contacting the RNA-binding protein with a circular RNA, wherein the circular RNA comprises a binding domain having a nucleotide sequence to which one or more copies of the RNA-binding protein binds. Preferably, binding of the RNA-binding protein to the circular RNA inhibits the metabolic function/activity of the RNA-binding protein.

In one embodiment, the present invention is directed to a method of inhibiting the activity of an RNA-binding protein, which method comprises contacting the RNA-binding protein with a circular RNA, wherein the circular RNA comprises a binding domain having a nucleotide sequence to which one or more copies of the RNA-binding protein binds thereby inhibiting the metabolic activity of the RNA-binding protein.

In one embodiment, the present invention is directed to a method of forming a complex comprising a circular RNA and an RNA-binding protein, which method comprises contacting the circular RNA with an RNA-binding protein, wherein the circular RNA comprises a binding domain having a nucleotide sequence to which one or more copies of the RNA-binding protein binds. Preferably, the RNA-binding protein in the complex is unable to perform its metabolic function/activity.

In any of the foregoing methods, the contacting can take place either *in vitro* or *in vivo.*

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., (1995) Helvetica Chimica Acta, CH-4010 Basel, Switzerland.

The practice of the present invention will employ, unless otherwise indicated, conventional methods of biochemistry, cell biology, immunology, and recombinant DNA techniques which are explained in the literature in the field *(e.g.,* Green and Sambrook, Molecular Cloning: A Laboratory Manual, 4th Edition, Cold Spring Harbor Laboratory Press, Cold Spring Harbor 2012).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, *i.e.,* the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (*e.g.,* "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, *etc*.), whether *supra* or *infra,* are hereby incorporated by reference in their entirety. Nothing herein is to be construed as an admission that the invention is not entitled to antedate such disclosure by virtue of prior invention.

The present invention is directed to a circular RNA molecule to which one or more copies of an RNA-binding protein binds, thereby allowing for the capture/sequestering of the RNA-binding protein in order to inhibit its function and methods for the use of such circular RNA molecules in therapeutic and non-therapeutic settings. The present invention is based, at least in part, on the inventors novel and unexpected finding that circular RNA molecules (circRNAs) can act as protein sponges, *i.e.,* that they can specifically bind and functionally inactivate RNA-binding proteins.

In one embodiment, the present invention is directed to a circular RNA molecule, which comprises a binding domain having a nucleotide sequence to which one or more copies of an RNA-binding protein binds. Preferably, the binding results in inhibiting the function/activity of the RNA-binding protein. Therefore, the circular RNA molecules described herein are particularly suited for use in treating or preventing diseases or disorders where inhibition of the activity of an RNA-binding protein is desired. For example, since RNA-binding proteins are involved in cellular processes necessary for the growth of the cell, RNA-binding proteins can be targeted by a circular RNA of the invention in order to inhibit the activity of the RNA-binding protein and, thus, inhibit cell growth. In this way, diseases and disorders characterized by unwanted cell growth, such as cancer, can be treated or prevented. This is particular pertinent in situations where overexpression (or increased activity) of an RNA-binding protein is indicative of a disease or disorder, such as cancer. Thus, a circular RNA to which this overexpressed RNA-binding protein binds can be used in methods to treat or prevent the disease or disorder by capturing and inhibiting the activity of this RNA-binding protein.

Exemplary RNA-binding proteins described herein include heterogeneous nuclear ribonucleoprotein L (hnRNP L), cold inducible RNA-binding protein (CIRBP), STAU1, STAU2, Sam68, FXR1, BYSL, ESRP1, CELF3, RBM24, IGF2BP2, IGF2BP3, NSUN6, ZC3H13, ELAC1, RBMS3, ZGPAT, SF3B1, SRSF2, RBM10, U2AF1, SF3B1, PPRC1, RBMXL1, and HNRNPCL1, and other hnRNP proteins (for a comprehensive list of RNA-binding proteins with known RNA-binding specificities, see Ray et al., 2013, Nature 499(7457): 172-177.

In a preferred embodiment of the present invention, the RNA-binding protein is heterogeneous nuclear ribonucleoprotein L (hnRNP L). Thus, the nucleotide sequence of the binding domain for a circular RNA that can bind one or more copies of hnRNP L comprises CA or AC dinucleotides. Preferably, the number of CA or AC dinucleotides is at least 20, at least 30, at least 40, at least 50, at least 60, at least 75, at least 100, at least 125, at least 150, at least 175, at least 200, at least 250, at least 300, at least 350, at least 400, or at least 450 CA or AC dinucleotides. In a specific embodiment, the number of CA or AC dinucleotides is at least 90 CA or AC dinucleotides. Preferably, at least 4 of the CA or AC dinucleotides are consecutive.

In preferred embodiments, the total nucleotide sequence of the circular RNA is less than 2000 nucleotides, less than 1500 nucleotides, less than 1250 nucleotides, less than 1000 nucleotides, less than 500 nucleotides, or less than 250 nucleotides in length. In a particularly preferred embodiment, the circular RNA molecule comprises the nucleotide sequence (with the t residues replaced with u residues) (SEQ ID NO: 1):

cacacacacacacacacacacacacacacacacacacacacacacacacacacacacacacacagatccacac

gcacacacacacacacacacacacacacacacacacacacacacacacacacacacacacagatccacacacg

acacacacagatccacacacacacacacacacacacacacacacacacacacacacacacacacacacacac

acaca.

In a preferred embodiment, the circular RNA molecule comprises the nucleotide sequence (with the T/t residues replaced with U/u residues) (SEQ ID NO:2):

AACCATGCCGAGTGCGGCCGCGGATCCatacatgacacacacacgcaatatatacatg
acacacacacgcaatatatacatgacacacacacgcaatatatacatgacacacacacgcaAGATGTGG
CCGCGGTCGGCGTGGACTGTAG

In a preferred embodiment, the circular RNA molecule comprises the nucleotide sequence (with the T/t residues replaced with U/u residues) (SEQ ID NO:3):

AAAATCCGTTGACCTTAAACGGTCGTGTGGGTTCAAGTCCCTCCACC
CCCACGCCGGAAACGCAATAGCCGAAAAACAAAAAACAAAAAAG
AGGATCcacacacacacacacacacacacacacacacacacacacacacacacacacaca
gatccacacgcacacacacacacacacacacacacacacacacacacacacacacacagatc
cacacacgacacacacagatccacacacacacacacacacacacacacacacacacacaca
cacacacacacacaAGATCCACTAGTAACGGCTCGAGAAAAACAAAAAA
CAAAACGGCTATTATGCGTTACCGGCGAGACGCTACGGACTT

In a preferred embodiment, the circular RNA molecule comprises the nucleotide sequence (with the T/t residues replaced with U/u residues) (SEQ ID NO:4):

GGGAGACCCAAGCTTCCGTCGATTGTCCACTGGTCAACAATAGATG
ACTTACAACTAATCGGAAGGTGCAGAGACTCGACGGAGCTACCCT
AACGTCAAGACGAGGGTAAAGAGAGAGTCCAATTCTCAAAGCCAA
TAGGCAGTAGCGAAAGCTGCAAGAGAATGAAAATCCGTTGACCTTA
AACGGTCGTGTGGGTTCAAGTCCCTCCACCCCCACGCCGGAAACGC
AATAGCCGAAAAACAAAAAACAAAAAAAGAGGATCcacacacacacacaca
cacacacacacacacacacacacacacacacacacacacacacagatccacacgcacacacacacacac
acacacacacacacacacacacacacacacacacacacacacagatccacacacgacacacacagatccaca
cacacacacacacacacacacacacacacacacacacacacacacacacacacacacaAGATCCA
CTAGTAACGGCTCGAGAAAAAACAAAAAACAAAACGGCTATTATG
CGTTACCGGCGAGACGCTACGGACTTAAATAATTGAGCCTTAAAGA

AGAAATTCTTTAAGTGGATGCTCTCAAACTCAGGGAAACCTAAATC
TAGTTATAGACAAGGCAATCCTGAGCCAAGCCGAAGTAGTAATTAG
TAAGACCAGTGGACAATCGACGGATAACAGCATATCTAGA

In a preferred embodiment, the circular RNA molecule comprises the nucleotide sequence (SEQ ID NO:5): GGGAGUAAGC-(CA)io-GCUUACAGUA. In a preferred embodiment, the circular RNA molecule comprises the nucleotide sequence (SEQ ID NO:6): GGGAGUAAGC-(CA)₁₅-GCUUACAGUA. In a preferred embodiment, the circular RNA molecule comprises the nucleotide sequence (SEQ ID NO:7): GGGAGUAAGC-(CA)₂₀-GCUUACAGUA. In a preferred embodiment, the circular RNA molecule comprises the nucleotide sequence (SEQ ID NO:8): GGGAGUAAGCAUACAUGACACACACACGCAGCUUACAGUA.

As disclosed herein, "nucleic acid sequence" refers to the sequence of nucleotides in a nucleic acid, *e.g.,* a ribonucleic acid (RNA) or a deoxyribonucleic acid (DNA). The term may refer to an entire nucleic acid molecule (such as to the single strand of an entire nucleic acid molecule) or to a part (*e.g.,* a fragment) thereof.

The term "recombinant" in the context of the present invention means "made through genetic engineering". Preferably, a "recombinant object" in the context of the present invention is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring. The term "found in nature" means "present in nature" and includes known objects as well as objects that have not yet been discovered and/or isolated from nature, but that may be discovered and/or isolated in the future from a natural source.

According to the invention, "double-stranded RNA" or "dsRNA" means RNA with two partially or completely complementary strands. Although the circRNA described herein preferably is a circularized "single-stranded" RNA molecule, it may nonetheless contain self-complementary sequences that allow parts of the RNA to fold back and to form secondary structure motifs including without limitation base pairs, stems, stem loops and bulges.

In the context of the present invention, the term "RNA" relates to a molecule which comprises ribonucleotide residues and preferably being entirely or substantially composed of ribonucleotide residues. "Ribonucleotide" relates to a nucleotide with a hydroxyl group at the 2'-position of a β-D-ribofuranosyl group. The term includes double-stranded RNA, single-stranded RNA, isolated RNA such as partially or completely purified RNA, essentially pure RNA, synthetic RNA, recombinantly produced RNA, as well as modified RNA that differs from naturally occurring RNA by the addition, deletion, substitution and/or alteration of one or more nucleotides. Such alterations can include addition of non-nucleotide material, such as internally, for example, at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA.

According to the present invention, the term "RNA" includes and preferably relates to "circular RNA" or "circRNA" which means an RNA molecule that has been circularized such that it does not have a 5' and 3' end, for example, one that results from the ligation of the 5' end to the 3' end or one that results from the action of one or more ribozymes. Preferably, the RNA is produced by *in vitro* transcription using a DNA template and then the ends are ligated together. In one embodiment of the invention, the RNA is obtained by *in vitro* transcription or chemical synthesis. The *in vitro* transcription and ligation methodologies are known to the skilled person. For example, there is a variety of commercially available *in vitro* transcription kits as well as ligation kits.

The RNA according to the invention may contain a modification, for example, a backbone modification, a sugar modification or a base modifications. In one embodiment, the RNA has modified ribonucleotides, which can increase its stability and/or decrease cytotoxicity or immunogenicity. For example, in one embodiment, in the RNA used according to the invention, 5-methylcytidine is substituted partially or completely, preferably completely, for cytidine. Alternatively or additionally, in one embodiment, in the RNA used according to the invention pseudouridine is substituted partially or completely, preferably completely, for uridine.

Other modified ribonucleotides include 1-methyl-adenine, 2-methyladenine, 2-methylthio-N6-isopentenyladenine, N6-methyladenine, N6-isopentenyladenine, 2-thiocytosine, 3-methylcytosine, 4-acetylcytosine, 5-methylcytosine, 2,6-diaminopurine, 1-methylguanine, 2-methylguanine, 2,2-dimethylguanine, 7-methylguanine, 7-deazaguanosine, inosine, 1-methylinosine, pseudouracil (5-uracil), dihydrouracil, 2-thiouracil, 4-thiouracil, 5-carboxymethylaminomethyl-2-thiouracil, 5-(carboxyhydroxymethyl)-uracil, 5-fluorouracil, 5-bromouracil, 5-carboxymethylaminomethyluracil, 5- methyl-2-thiouracil, 5-methyluracil, N-uracil-5-oxyacetic acid methyl ester, 5-methylaminomethyluracil, 5-methoxyaminomethyl-2-thiouracil, 5'-methoxycarbonylmethyluracil, 5-methoxyuracil, uracil-5-oxyacetic acid methyl ester, uracil-5-oxyacetic acid, 1-methylpseudouracil, queosine, and β-D-mannosylqueosine.

Backbone modifications typically include, without being limited thereto, modifications from the group consisting of methylphosphonates, methylphosphoramidates, phosphoramidates, phosphorothioates, and boranophosphates.

A sugar modification in connection with the circular RNA described herein is a chemical modification of the sugar of the nucleotides it contains and typically includes, without being limited thereto, sugar modifications chosen from the group consisting of 2'-deoxy-2'-fluoro-oligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5 '-triphosphate).

The term "stability" of an agent, *e.g.,* circRNA, to the "half-life" of the agent. "Half-life" relates to the period of time which is needed to eliminate half of the maximum (pharmacologic) activity, amount, or number of molecules of an agent, *e.g.,* from the body or blood of a patient to which the agent was administered or as measured in an *in vitro* assay. The maximum pharmacologic activity is defined by the steady state value, where intake equals elimination. Since the kinetics of certain agents, such as pharmaceutical drugs, can be complex, the "half-life" of an agent does not necessarily follow or is limited to first order kinetics.

The RNA to be administered according to the invention is preferably non-immunogenic. The term "non-immunogenic RNA" as used herein refers to RNA that does not induce a response by the immune system upon administration, *e.g*., to a mammal, or induces a weaker response than would have been induced by the same RNA that differs only in that it has not been subjected to the modifications and treatments that render the non-immunogenic RNA non-immunogenic. In one preferred embodiment non-immunogenic RNA is rendered non-immunogenic by incorporating modified nucleotides suppressing RNA-mediated activation of innate immune receptors into the RNA or by having a sequence that does not form double-stranded structures or by removing any double-stranded RNA (dsRNA) present with the circRNA.

For rendering the non-immunogenic RNA non-immunogenic by the incorporation of modified nucleotides, any modified nucleotide may be used as long as it lowers or suppresses immunogenicity of the RNA. Particularly preferred are modified nucleotides that suppress RNA-mediated activation of innate immune receptors. In one embodiment, the modified nucleotides comprises a replacement of one or more uridines with a nucleoside comprising a modified nucleobase. In one embodiment, the modified nucleobase is a modified uracil. In one embodiment, the nucleoside comprising a modified nucleobase is selected from the group consisting of 3-methyl-uridine (m3U), 5-methoxy-uridine (mo5U), 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho5U), 5-aminoallyl-uridine, 5-halo-uridine (*e.g*., 5-iodo-uridineor 5-bromo-uridine), uridine 5-oxyacetic acid (cmo5U), uridine 5-oxyacetic acid methyl ester (mcmo5U), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm5U), 5-methoxycarbonylmethyl-2-thio-uridine (mcm5s2U), 5-aminomethyl-2-thio-uridine (nm5s2U), 5-methylaminomethyl-uridine (mnm5U), 1-ethyl-pseudouridine, 5-methylaminomethyl-2-thio-uridine (mnm5s2U), 5-methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyl-uridine, 1-propynyl-pseudouridine, 5-taurinomethyl-uridine (τm5U), 1-taurinomethyl-pseudouridine, 5-taurinomethyl-2-thio-uridine (τm5s2U), 1-taurinomethyl-4-thio-pseudouridine, 5-methyl-2-thio-uridine (m5s2U), 1-methyl-4-thio-pseudouridine (m1s4ψ), 4-thio-1-methyl-pseudouridine, 3-methyl-pseudouridine (m3ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m5D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp3U), 1-methyl-3-(3-amino-3-carboxypropyl)pseudouridine (acp3 ψ), 5-(isopentenylaminomethyl)uridine (inm5U), 5-(isopentenylaminomethyl)-2-thio-uridine (inm5s2U), α-thio-uridine, 2'-O-methyl-uridine (Um), 5,2'-O-dimethyl-uridine (m5Um), 2'-O-methyl-pseudouridine (ψm), 2-thio-2'-O-methyl-uridine (s2Um), 5-methoxycarbonylmethyl-2'-O-methyl-uridine (mcm5Um), 5-carbamoylmethyl-2'-O-methyl-uridine (ncm5Um), 5-carboxymethylaminomethyl-2'-O-methyl-uridine (cmnm5Um), 3,2'-O-dimethyl-uridine (m3Um), 5-(isopentenylaminomethyl)-2'-O-methyl-uridine (inm5Um), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino)uridine. In one particularly preferred embodiment, the nucleoside comprising a modified nucleobase is pseudouridine (ψ), N1-methyl-pseudouridine (m1ψ) or 5-methyl-uridine (m5U), in particular 1-methyl-pseudouridine.

The structure of an exemplary nucleoside comprising a modified nucleobase is 1-methylpseudouridine m1Ψ:

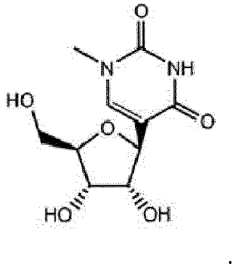

In one embodiment, the replacement of one or more uridines with a nucleoside comprising a modified nucleobase comprises a replacement of at least 1%, at least 2%, at least 3%, at least 4%, at least 5%, at least 10%, at least 25%, at least 50%, at least 75%, at least 90%, at least 95%, at least 96%, at least 97%, at least 98%, at least 99% or 100% of the uridines.

During synthesis of mRNA by *in vitro* transcription (IVT) using T7 RNA polymerase significant amounts of aberrant products, including double-stranded RNA (dsRNA) are produced due to unconventional activity of the enzyme. dsRNA induces inflammatory cytokines and activates effector enzymes leading to protein synthesis inhibition. dsRNA can be removed from RNA such as IVT RNA, for example, by ion-pair reversed phase HPLC using a non-porous or porous C-18 polystyrene-divinylbenzene (PS-DVB) matrix. Alternatively, an enzymatic based method using E. coli RNaseIII that specifically hydrolyzes dsRNA but not ssRNA, thereby eliminating dsRNA contaminants from IVT RNA preparations can be used. Furthermore, dsRNA can be separated from ssRNA by using a cellulose material. In one embodiment, an RNA preparation is contacted with a cellulose material and the ssRNA is separated from the cellulose material under conditions which allow binding of dsRNA to the cellulose material and do not allow binding of ssRNA to the cellulose material. It is to be understood that the unwanted dsRNA can be removed prior to or after the ligation step forming the circular RNA.

As the term is used herein, "remove" or "removal" refers to the characteristic of a population of first substances, such as non-immunogenic RNA, being separated from the proximity of a population of second substances, such as dsRNA, wherein the population of first substances is not necessarily devoid of the second substance, and the population of second substances is not necessarily devoid of the first substance. However, a population of first substances characterized by the removal of a population of second substances has a measurably lower content of second substances as compared to the non-separated mixture of first and second substances.

Nucleic acids can be transferred into a host cell by physical, chemical or biological means. Physical methods for introducing a nucleic acid into a host cell include calcium phosphate precipitation, lipofection, particle bombardment, microinjection, electroporation, and the like.

Biological methods for introducing a nucleic acid of interest into a host cell include the use of DNA and RNA vectors. Chemical means for introducing a nucleic acid into a host cell include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (*i.e.,* an artificial membrane vesicle). The preparation and use of such systems is well known in the art.

The term "cell" or "host cell" preferably is an intact cell, *i.e.,* a cell with an intact membrane that has not released its normal intracellular components such as enzymes, organelles, or genetic material. An intact cell preferably is a viable cell, *i.e.,* a living cell capable of carrying out its normal metabolic functions. Preferably said term relates according to the invention to any cell which can be transformed or transfected with an exogenous nucleic acid. The term "cell" includes according to the invention prokaryotic cells (*e.g.,* E. coli) or eukaryotic cells (*e.g.,* dendritic cells, B cells, CHO cells, COS cells, K562 cells, HEK293 cells, HeLa cells, yeast cells, and insect cells). Mammalian cells are particularly preferred, such as cells from humans, mice, hamsters, pigs, goats, and primates. The cells may be derived from a large number of tissue types and include primary cells and cell lines.

Terms such as "reducing", "inhibiting" or "decreasing" relate to the ability to cause an overall decrease, preferably of 5% or greater, 10% or greater, 20% or greater, more preferably of 50% or greater, and most preferably of 75% or greater, in the level. These terms include a complete or essentially complete inhibition, *i.e.* a reduction to zero or essentially to zero.

Terms such as "increasing", "enhancing", "promoting", "stimulating", or "inducing" relate to the ability to cause an overall increase, preferably of 5% or greater, 10% or greater, 20% or greater, 50% or greater, 75% or greater, 100% or greater, 200% or greater, or 500% or greater, in the level. These terms may relate to an increase, enhancement, promotion, stimulation, or inducement from zero or a non-measurable or non-detectable level to a level of more than zero or a level which is measurable or detectable. Alternatively, these terms may also mean that there was a certain level before an increase, enhancement, promotion, stimulation, or inducement and after the increase, enhancement, promotion, stimulation, or inducement the level is higher.

The term "isolated" as used herein, is intended to refer to a molecule which is substantially free of other molecules such as other cellular material. The term "isolated nucleic acid" means according to the invention that the nucleic acid has been (i) amplified *in vitro,* for example by polymerase chain reaction (PCR), (ii) recombinantly produced by cloning, (iii) purified, for example by cleavage and gel-electrophoretic fractionation, or (iv) synthesized, for example by chemical synthesis. An isolated nucleic acid is a nucleic acid available to manipulation by recombinant techniques.

The circular RNA and compositions described herein are useful in methods of inhibiting the activity of an RNA-binding protein, for example, for the treatment or prevention of a disease or disorder characterized by unwanted cell growth. According to the invention circRNA, preferably non-immunogenic circRNA, is administered to the patient/subject.

The term "disease" refers to an abnormal condition that affects the body of an individual. A disease is often construed as a medical condition associated with specific symptoms and signs. A disease may be caused by factors originally from an external source, such as infectious disease, or it may be caused by internal dysfunctions, such as cancer. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality. According to the invention, the term "disease" preferably includes hyperproliferative disorders, such as cancer.

The terms "cancer disease" or "cancer" refer to or describe the physiological condition in an individual that is typically characterized by unregulated cell growth. Examples of cancers include, but are not limited to, carcinoma, lymphoma, blastoma, sarcoma, and leukemia. More particularly, examples of such cancers include bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma. The term "cancer" according to the invention also comprises cancer metastases.

A disease to be treated according to the invention is preferably a disease involving unwanted cell growth, *e.g.,* cancer. Other diseases include those that can be treated by inhibition of the activity of an RNA-binding protein using a circular RNA of the present invention.

The term "treatment" or "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual, in particular an individual being at risk for the disease. The terms "prophylactic treatment" or "preventive treatment" are used herein interchangeably.

The terms "protect", "prevent", "prophylactic", "preventive", or "protective" relate to the prevention and/or treatment of the occurrence and/or the propagation of a disease, *e.g*., tumor, in an individual. For example, a prophylactic administration of a circRNA, *e.g.,* by administering a composition of the present invention, can protect the receiving individual from the development of a tumor. For example, by administering a composition of the present invention, the development of a disease can be stopped, *e.g*., leading to inhibition of the progress/growth of a tumor. This comprises the deceleration of the progress/growth of the tumor, in particular a disruption of the progression of the tumor, which preferably leads to elimination of the tumor.

By "being at risk" is meant a subject, *i.e.,* a patient, that is identified as having a higher than normal chance of developing a disease compared to the general population. In addition, a subject who has had, or who currently has, a disease is a subject who has an increased risk for developing a disease, as such a subject may continue to develop a disease.

The term *"in vivo"* relates to the situation in a subject.

The term "individual" or "subject" or "patient" relates to vertebrates, particularly mammals. For example, mammals in the context of the present invention are humans, non-human primates, domesticated mammals such as dogs, cats, sheep, cattle, goats, pigs, horses *etc.,* laboratory animals such as mice, rats, rabbits, guinea pigs, *etc.* as well as animals in captivity such as animals of zoos. The term "subject" also relates to non-mammalian vertebrates such as birds (particularly domesticated birds such as chicken, ducks, geese, turkeys) and to fish (particularly farmed fish, *e.g.* salmon or catfish). The term "animal" as used herein also includes humans.

The agents, *e.g.,* the circular RNA molecules described herein, may be administered in the form of any suitable pharmaceutical composition. The term "pharmaceutical composition" relates to a formulation comprising a therapeutically effective agent or a salt thereof, preferably together with pharmaceutical excipients such as buffers, preservatives and tonicity modifiers. Said pharmaceutical composition is useful for treating or preventing a disease or disorder by administration of said pharmaceutical composition to an individual. A pharmaceutical composition is also known in the art as a pharmaceutical formulation. The pharmaceutical composition can be administered locally or systemically.

The term "systemic administration" refers to the administration of a therapeutically effective agent such that the agent becomes widely distributed in the body of an individual in significant amounts and develops a biological effect. According to the present invention, it is preferred that administration is by parenteral administration.

The term "parenteral administration" refers to administration of a therapeutically effective agent such that the agent does not pass the intestine. The term "parenteral administration" includes intravenous administration, subcutaneous administration, intradermal administration or intraarterial administration but is not limited thereto.

The pharmaceutical composition according to the present invention is generally applied in a "pharmaceutically effective amount" and in "a pharmaceutically acceptable preparation".

The term "pharmaceutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severity of the disease, the individual parameters of the patient, including age, physiological condition, size and weight, the duration of treatment, the type of an accompanying therapy (if present), the specific route of administration and similar factors. Accordingly, the doses administered of the compositions described herein may depend on several of such parameters. In the case that a reaction in a patient is insufficient with an initial dose, higher doses (or effectively higher doses achieved by a different, more localized route of administration) may be used.

The term "pharmaceutically acceptable" refers to the non-toxicity of a material which does not interact with the action of the active component of the pharmaceutical composition.

The pharmaceutical compositions of the present invention may contain salts, buffers, preserving agents, carriers and optionally other therapeutic agents. Preferably, the pharmaceutical compositions of the present invention comprise one or more pharmaceutically acceptable carriers, diluents and/or excipients.

The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents or encapsulating substances, which are suitable for administration to a patient. Possible carrier substances include, *e.g.,* sterile water, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxypropylene copolymers, oils, including those which are derived from mineral oil, animals, or plants, such as peanut oil, soy bean oil, sesame oil, sunflower oil, *etc.* Salt solutions and aqueous dextrose and glycerin solutions may also be used as aqueous carrier compounds.

The term "excipient" when used herein is intended to indicate all substances which may be present in a pharmaceutical composition and which are not active ingredients such as, *e.g.,* carriers, binders, lubricants, thickeners, surface active agents, preservatives, emulsifiers, buffers, flavoring agents, or colorants.

The term "diluent" relates a diluting and/or thinning agent. Moreover, the term "diluent" includes any one or more of fluid, liquid or solid suspension and/or mixing media.

Pharmaceutically acceptable carriers or diluents for therapeutic use are well known in the pharmaceutical art, and are described, for example, in Remington's Pharmaceutical Sciences, Mack Publishing Co. (A. R Gennaro edit. 1985). Examples of suitable carriers include, for example, magnesium carbonate, magnesium stearate, talc, sugar, lactose, pectin, dextrin, starch, gelatin, tragacanth, methylcellulose, sodium carboxymethylcellulose, a low-melting wax, cocoa butter, and the like. Examples of suitable diluents include ethanol, glycerol and water.

Pharmaceutical carriers, excipients or diluents can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavoring agents may be provided in the pharmaceutical composition. Examples of preservatives include sodium benzoate, benzalkonium chloride, chlorobutanol, paraben, thimersol, sorbic acid and esters of p-hydroxybenzoic acid. Antioxidants and suspending agents may be also used.

In one embodiment, the composition is an aqueous composition. The aqueous composition may optionally comprise solutes, *e.g*., salts. In one embodiment, the composition is in the form of a freeze-dried composition. A freeze-dried composition is obtainable by freeze-drying a respective aqueous composition.

In one embodiment, the composition is an injectable composition, optionally comprising sodium, potassium and calcium salts, such as Ringer's solution or Ringer's Lactate.

Terms such as "transferring", "introducing", "transfecting" or "transducing" are used interchangeably herein and relate to the introduction of nucleic acids, in particular exogenous or heterologous nucleic acids, such as circRNA into a cell. According to the present invention, the cell can be present *in vitro* or *in vivo, e.g.,* the cell can form part of an organ, a tissue and/or an organism. According to the invention, transfection can be transient or stable. For some applications of transfection, it is sufficient if the transfected genetic material is only transiently present. Since the nucleic acid introduced in the transfection process is usually not integrated into the nuclear genome, the foreign nucleic acid will be diluted through mitosis or degraded.

In one embodiment, the circRNA molecule to be administered is naked, *i.e.,* not complexed with any kind of delivery material or proteins. In one embodiment, the RNA molecule to be administered is formulated in a delivery vehicle. In one embodiment, the delivery vehicle comprises particles. In one embodiment, the delivery vehicle comprises a lipid. In one embodiment, the lipid comprises a cationic lipid. In one embodiment, the lipid forms a complex with and/or encapsulates the RNA molecule. In one embodiment, the RNA molecule is formulated in liposomes.

The pharmaceutical compositions of the invention are preferably sterile and contain an effective amount of the agents described herein and optionally of further agents as discussed herein to generate the desired reaction or the desired effect.

Pharmaceutical compositions are usually provided in a uniform dosage form and may be prepared in a manner known *per se.* A pharmaceutical composition may, *e.g.,* be in the form of a solution or suspension.

In one embodiment, if the pharmaceutical composition comprises nucleic acids, it can comprise at least one cationic entity. In general, cationic lipids, cationic polymers and other substances with positive charges may form complexes with negatively charged nucleic acids. It is possible to stabilize the RNA according to the invention by complexation with cationic compounds, preferably polycationic compounds such as for example a cationic or polycationic peptide or protein. In one embodiment, the pharmaceutical composition according to the present invention comprises at least one cationic molecule selected from the group consisting protamine, polyethylene imine, a poly-L-lysine, a poly-L-arginine, a histone or a cationic lipid.

According to the present invention, a cationic lipid is a cationic amphiphilic molecule, *e.g.,* a molecule which comprises at least one hydrophilic and lipophilic moiety. The cationic lipid can be monocationic or polycationic. Cationic lipids typically have a lipophilic moiety, such as a sterol, an acyl or diacyl chain, and have an overall net positive charge. The head group of the lipid typically carries the positive charge. The cationic lipid preferably has a positive charge of 1 to 10 valences, more preferably a positive charge of 1 to 3 valences, and more preferably a positive charge of 1 valence. Examples of cationic lipids include, but are not limited to 1,2-di-O-octadecenyl-3-trimethylammonium propane (DOTMA); dimethyldioctadecylammonium (DDAB); 1,2-dioleoyl-3-trimethylammonium-propane (DOTAP); 1,2-dioleoyl-3-dimethylammonium-propane (DODAP); 1,2-diacyloxy-3-dimethylammonium propanes; 1,2-dialkyloxy-3-dimethylammonium propanes; dioctadecyldimethyl ammonium chloride (DODAC), 1,2-dimyristoyloxypropyl-1,3-dimethylhydroxyethyl ammonium (DMRIE), and 2,3-dioleoyloxy-N-[2(spermine carboxamide)ethyl]-N,N-dimethyl-1-propanamium trifluoroacetate (DOSPA). Cationic lipids also include lipids with a tertiary amine group, including 1,2-dilinoleyloxy-N,N-dimethyl-3-aminopropane (DLinDMA). Cationic lipids are suitable for formulating RNA in lipid formulations as described herein, such as liposomes, emulsions and lipoplexes. Typically positive charges are contributed by at least one cationic lipid and negative charges are contributed by the RNA. In one embodiment, the pharmaceutical composition comprises at least one helper lipid, in addition to a cationic lipid. The helper lipid may be a neutral or an anionic lipid. The helper lipid may be a natural lipid, such as a phospholipid, or an analogue of a natural lipid, or a fully synthetic lipid, or lipid-like molecule, with no similarities with natural lipids. In the case where a pharmaceutical composition includes both a cationic lipid and a helper lipid, the molar ratio of the cationic lipid to the neutral lipid can be appropriately determined in view of stability of the formulation and the like.

In one embodiment, the pharmaceutical composition according to the present invention comprises protamine. According to the invention, protamine is useful as cationic carrier agent. The term "protamine" refers to any of various strongly basic proteins of relatively low molecular weight that are rich in arginine and are found associated especially with DNA in place of somatic histones in the sperm cells of animals such as fish. In particular, the term "protamine" refers to proteins found in fish sperm that are strongly basic, are soluble in water, are not coagulated by heat, and comprise multiple arginine monomers. According to the invention, the term "protamine" as used herein is meant to comprise any protamine amino acid sequence obtained or derived from native or biological sources including fragments thereof and multimeric forms of said amino acid sequence or fragment thereof. Furthermore, the term encompasses (synthesized) polypeptides which are artificial and specifically designed for specific purposes and cannot be isolated from native or biological sources.

The pharmaceutical composition according to the invention can be buffered, (*e.g*., with an acetate buffer, a citrate buffer, a succinate buffer, a Tris buffer, a phosphate buffer).

In some embodiments, the circRNA molecules of the present invention are provided in complexed or encapsulated form. Respective pharmaceutical compositions are provided in the present invention. In particular, in some embodiments, the pharmaceutical composition of the present invention comprises nucleic acid-containing particles, preferably RNA-containing particles. Respective pharmaceutical compositions are referred to as particulate formulations. In particulate formulations according to the present invention, a particle comprises nucleic acid according to the invention and a pharmaceutically acceptable carrier or a pharmaceutically acceptable vehicle that is suitable for delivery of the nucleic acid. The nucleic acid-containing particles may be, for example, in the form of proteinaceous particles or in the form of lipid-containing particles. Suitable proteins or lipids are referred to as particle-forming agents. Proteinaceous particles and lipid-containing particles have been described previously to be suitable for delivery of RNA in particulate form (*e.g.,* Strauss & Strauss, 1994, Microbiol. Rev. 58:491-562).

In one embodiment, the particulate formulation of the present invention is a nanoparticulate formulation. In that embodiment, the composition according to the present invention comprises nucleic acid according to the invention in the form of nanoparticles. Nanoparticulate formulations can be obtained by various protocols and with various complexing compounds. Lipids, polymers, oligomers, or amphipiles are typical constituents of nanoparticulate formulations.

As used herein, the term "nanoparticle" refers to any particle having a diameter making the particle suitable for systemic, in particular parenteral, administration, of, in particular, nucleic acids, typically a diameter of 1000 nanometers (nm) or less. In one embodiment, the nanoparticles have an average diameter in the range of from about 50 nm to about 1000 nm, preferably from about 50 nm to about 400 nm, preferably about 100 nm to about 300 nm such as about 150 nm to about 200 nm. In one embodiment, the nanoparticles have a diameter in the range of about 200 to about 700 nm, about 200 to about 600 nm, preferably about 250 to about 550 nm, in particular about 300 to about 500 nm or about 200 to about 400 nm.

In one embodiment, the polydispersity index (PI) of the nanoparticles described herein, as measured by dynamic light scattering, is 0.5 or less, preferably 0.4 or less or even more preferably 0.3 or less. The "polydispersity index" (PI) is a measurement of homogeneous or heterogeneous size distribution of the individual particles (such as liposomes) in a particle mixture and indicates the breadth of the particle distribution in a mixture. The PI can be determined, for example, as described in WO 2013/143555 A1.

As used herein, the term "nanoparticulate formulation" or similar terms refer to any particulate formulation that contains at least one nanoparticle. In some embodiments, a nanoparticulate composition is a uniform collection of nanoparticles. In some embodiments, a nanoparticulate composition is a lipid-containing pharmaceutical formulation, such as a liposome formulation or an emulsion.

In one embodiment, the pharmaceutical composition according to the invention comprises circRNA encapsulated in a vesicle, *e.g.,* in a liposome. In one embodiment, the pharmaceutical composition according to the invention comprises circRNA in the form of an emulsion. In one embodiment, the pharmaceutical composition according to the invention comprises circRNA in a complex with a cationic compound, thereby forming, *e.g.,* so-called lipoplexes or polyplexes. Encapsulation of RNA within vesicles such as liposomes is distinct from, for instance, lipid/RNA complexes. Lipid/RNA complexes are obtainable, *e.g.,* when RNA is, *e.g.,* mixed with pre-formed liposomes.

In one embodiment, the pharmaceutical composition according to the invention comprises circRNA encapsulated in a vesicle. Such formulation is a particular particulate formulation according to the invention. A vesicle is a lipid bilayer rolled up into a spherical shell, enclosing a small space and separating that space from the space outside the vesicle. Typically, the space inside the vesicle is an aqueous space, *i.e.,* comprises water. Typically, the space outside the vesicle is an aqueous space, *i.e.,* comprises water. The lipid bilayer is formed by one or more lipids (vesicle-forming lipids). The membrane enclosing the vesicle is a lamellar phase, similar to that of the plasma membrane. The vesicle according to the present invention may be a multilamellar vesicle, a unilamellar vesicle, or a mixture thereof. When encapsulated in a vesicle, the circRNA is typically separated from any external medium. Suitable vesicles are particles, particularly nanoparticles, as described herein.

For example, circRNA may be encapsulated in a liposome. In that embodiment, the pharmaceutical composition is or comprises a liposome formulation. Encapsulation within a liposome will typically protect RNA from RNase digestion. It is possible that the liposomes include some external RNA (*e.g.,* on their surface), but at least half of the RNA (and ideally all of it) is encapsulated within the core of the liposome.

Liposomes are microscopic lipidic vesicles often having one or more bilayers of a vesicle-forming lipid, such as a phospholipid, and are capable of encapsulating a drug, *e.g.,* circRNA. Different types of liposomes may be employed in the context of the present invention, including, without being limited thereto, multilamellar vesicles (MLV), small unilamellar vesicles (SUV), large unilamellar vesicles (LUV), sterically stabilized liposomes (SSL), multivesicular vesicles (MV), and large multivesicular vesicles (LMV) as well as other bilayered forms known in the art. The size and lamellarity of the liposome will depend on the manner of preparation. There are several other forms of supramolecular organization in which lipids may be present in an aqueous medium, comprising lamellar phases, hexagonal and inverse hexagonal phases, cubic phases, micelles, reverse micelles composed of monolayers.

Liposomes may be formed using standard methods known to the skilled person. Respective methods include the reverse evaporation method, the ethanol injection method, the dehydration-rehydration method, sonication or other suitable methods. Following liposome formation, the liposomes can be sized to obtain a population of liposomes having a substantially homogeneous size range.

In one embodiment, the circRNA is present in a liposome which includes at least one cationic lipid. Respective liposomes can be formed from a single lipid or from a mixture of lipids, provided that at least one cationic lipid is used. Preferred cationic lipids have a nitrogen atom which is capable of being protonated; preferably, such cationic lipids are lipids with a tertiary amine group. A particularly suitable lipid with a tertiary amine group is 1,2-dilinoleyloxy-N,N-dimethyl-3-aminopropane (DLinDMA). In one embodiment, the RNA according to the present invention is present in a liposome formulation as described in WO 2012/006378 A1: a liposome having a lipid bilayer encapsulating an aqueous core including RNA, wherein the lipid bilayer comprises a lipid with a pKa in the range of 5.0 to 7.6, which preferably has a tertiary amine group. Preferred cationic lipids with a tertiary amine group include DLinDMA (pKa 5.8) and are generally described in WO 2012/031046 A2. According to WO 2012/031046 A2, liposomes comprising a respective compound are particularly suitable for encapsulation of RNA and thus liposomal delivery of RNA. In one embodiment, the circRNA according to the present invention is present in a liposome formulation, wherein the liposome includes at least one cationic lipid whose head group includes at least one nitrogen atom (N) which is capable of being protonated, wherein the liposome and the RNA have a N:P ratio of between 1:1 and 20:1. According to the present invention, "N:P ratio" refers to the molar ratio of nitrogen atoms (N) in the cationic lipid to phosphate atoms (P) in the RNA comprised in a lipid containing particle (*e.g.,* liposome), as described in WO 2013/006825 A1. The N:P ratio of between 1:1 and 20:1 is implicated in the net charge of the liposome and in efficiency of delivery of RNA to a vertebrate cell.

In one embodiment, the circRNA according to the present invention is present in a liposome formulation that comprises at least one lipid which includes a polyethylene glycol (PEG) moiety, wherein RNA is encapsulated within a PEGylated liposome such that the PEG moiety is present on the liposome's exterior, as described in WO 2012/031043 A1 and WO 2013/033563 A1.

In one embodiment, the circRNA according to the present invention is present in a liposome formulation, wherein the liposome has a diameter in the range of 60-180 nm, as described in WO 2012/030901 A1.

In one embodiment, the circRNA according to the present invention is present in a liposome formulation, wherein the RNA-containing liposomes have a net charge close to zero or negative, as disclosed in WO 2013/143555 A1.

In other embodiments, the circRNA according to the present invention is present in the form of an emulsion. Emulsions have been previously described to be used for delivery of nucleic acid molecules, such as RNA molecules, to cells. Preferred herein are oil-in-water emulsions. The respective emulsion particles comprise an oil core and a cationic lipid. More preferred are cationic oil-in-water emulsions in which the circRNA according to the present invention is complexed to the emulsion particles. The emulsion particles comprise an oil core and a cationic lipid. The cationic lipid can interact with the negatively charged RNA, thereby anchoring the circRNA to the emulsion particles. In an oil-in-water emulsion, emulsion particles are dispersed in an aqueous continuous phase. For example, the average diameter of the emulsion particles may typically be from about 80 nm to 180 nm. In one embodiment, the pharmaceutical composition of the present invention is a cationic oil-in-water emulsion, wherein the emulsion particles comprise an oil core and a cationic lipid, as described in WO 2012/006380 A2. The RNA according to the present invention may be present in the form of an emulsion comprising a cationic lipid wherein the N:P ratio of the emulsion is at least 4:1, as described in WO 2013/006834 A1. The RNA according to the present invention may be present in the form of a cationic lipid emulsion, as described in WO 2013/006837 A1. In particular, the composition may comprise RNA complexed with a particle of a cationic oil-in-water emulsion, wherein the ratio of oil/lipid is at least about 8:1 (mole:mole).

In other embodiments, the pharmaceutical composition according to the invention comprises circRNA in the format of a lipoplex. The term, "lipoplex" or "RNA lipoplex" refers to a complex of lipids and nucleic acids such as RNA. Lipoplexes can be formed of cationic (positively charged) liposomes and the anionic (negatively charged) nucleic acid. The cationic liposomes can also include a neutral "helper" lipid. In the simplest case, the lipoplexes form spontaneously by mixing the nucleic acid with the liposomes with a certain mixing protocol, however various other protocols may be applied. It is understood that electrostatic interactions between positively charged liposomes and negatively charged nucleic acid are the driving force for the lipoplex formation (WO 2013/143555 A1). In one embodiment of the present invention, the net charge of the RNA lipoplex particles is close to zero or negative. It is known that electro-neutral or negatively charged lipoplexes of RNA and liposomes lead to substantial RNA expression in spleen dendritic cells (DCs) after systemic administration and are not associated with the elevated toxicity that has been reported for positively charged liposomes and lipoplexes (see WO 2013/143555 A1). Therefore, in one embodiment of the present invention, the pharmaceutical composition according to the invention comprises RNA in the format of nanoparticles, preferably lipoplex nanoparticles, in which (i) the number of positive charges in the nanoparticles does not exceed the number of negative charges in the nanoparticles and/or (ii) the nanoparticles have a neutral or net negative charge and/or (iii) the charge ratio of positive charges to negative charges in the nanoparticles is 1.4:1 or less and/or (iv) the zeta potential of the nanoparticles is 0 or less. As described in WO 2013/143555 A1, zeta potential is a scientific term for electrokinetic potential in colloidal systems. In the present invention, (a) the zeta potential and (b) the charge ratio of the cationic lipid to the RNA in the nanoparticles can both be calculated as disclosed in WO 2013/143555 A1. In summary, pharmaceutical compositions which are nanoparticulate lipoplex formulations with a defined particle size, wherein the net charge of the particles is close to zero or negative, as disclosed in WO 2013/143555 A1, are preferred pharmaceutical compositions in the context of the present invention.

The compounds provided herein, *e.g.,* the circRNA molecules, and compositions comprising the compounds provided herein, may be used alone or in combination with conventional therapeutic regimens.

The present invention is further illustrated by the following figures and examples which are not be construed as limiting the scope of the invention.

### FIGURES

**Figure 1.** Specific and efficient binding of hnRNP L by designer sponge circRNAs.
   (**A**) Circular RNAs used for *in vitro* hnRNP L sponging (*in vitro* circularization site indicated by line; total sizes given in parentheses).
   (**B**) HnRNP L sponging *in vitro.* Short RNAs containing 10, 15, or 20 CA-dinucleotide repeats, a CA-rich, SELEX-derived RNA sequence, as well as a negative control RNA, each in linear (-) or circular (O) configuration, were synthesized in biotinylated form. In addition, a linear (CA)₃₂ RNA was used as a positive control. After incubation in nuclear extract from HeLa cells, hnRNP L binding was assayed by pulldown with streptavidin agarose, followed by Western blot analysis for bound hnRNP L, comparing supernatant (SN; 2.5%), bound material (B; 20%) and input (2.5%). GAPDH served as an additional input control.
   (C) HnRNP L sponging *in vivo.* Lysates were prepared from HEK293 cell lines stably expressing hnRNP L sponge circRNA (CA)₁₀₀ (top panel) or a negative control RNA (bottom panel), followed by immunoprecipitation (IP) with anti-hnRNP L, or as controls, with anti-IMP3 or -FLAG (mock) antibodies. For comparison, 5% of input lysates was applied. Immunoprecipitated circRNAs were detected by RT-PCR. M, 200/300/400/500 bp markers.
**Figure 2.** Transfected hnRNP L-sponge circRNAs regulate alternative splicing *in vivo.*
   (A) Synthesis of large circRNAs, based on the PIE self-splicing system (Wesselhoeft *et al.,* 2018, Nat Commun 9(1):2629). RNAs containing 100 CA-dinucleotides or a negative control, both in linear and circular configuration, were produced (see panels B and C) for transfection in mammalian cells. Alternatively, circRNA sponges can be produced *in vivo* from a transfected circRNA expression construct, here a SELEX-derived, CA-rich sequence, present in four copies within the circRNA (see panel D).
   (**B**) (CA)₁₀₀ RNA and control RNA were synthesized by T7 transcription (lin), followed by *in vitro* PIE-mediated processing to circular RNA (circ), RNase R digestion (-/+), and HPLC purification. Linear and circular monomer RNA species are marked. RNA was analyzed by E-gel electrophoresis and visualized by Sybr-Gold. *M*, markers (HR RNA ladder).
   (**C**) Alternative splicing changes after transfection of CA-sponge circRNA. (CA)₁₀₀ hnRNP L-sponge or control RNAs, either in linear or circular configuration (lin/circ), were transfected in HeLa cells (100 or 500 ng per transfection). After 24 hr, alternative splicing was assayed by RT-PCR for two known hnRNP L targets, *TJP1* and *FALZ,* where hnRNP L functions as a splice repressor. The two RT-PCR products indicate exon inclusion (red arrows) and skipping, respectively; quantitation of exon inclusion (in %) is indicated in the respective lanes.
   (**D**) Alternative splicing changes after expression of a CA-rich sequence-containing circRNA, CA-SELEX X4, which contains four copies of a SELEX-derived 20-mer binding sequence for hnRNP L. HeLa cells were transfected with the CA-SELEX X4 expression construct [as controls, left untransfected (untrf) or control-transfected (ctr) for two days], and after one, two, three and four days, alternative splicing was assayed by RT-PCR for two known hnRNP L targets, *TJP1* and *FALZ,* as described in panel C.
**Figure 3.** General concept and global analysis of sponging of RNA-binding proteins by designer circRNAs.
   (**A**) General concept of alternative splicing modulation induced by CA-sponges for hnRNP L. This is illustrated here for an example of exon skipping and inclusion, regulated by hnRNP L, which is known to act either as splicing activator or repressor. Such splicing decisions are modulated by a CA-sponge, which inactivates hnRNP L, resulting in a shift in the ratio of skipping versus inclusion isoforms, depending on whether hnRNP L acts as a repressor or activator.
   (**B**) Genomewide analysis of alternative splicing changes induced by circRNA-based hnRNP L-sponging: principle and summary of results. The CA-sponge effects were compared directly with classical RNAi-induced hnRNP L knockdown effects.
   (**C**) Validation of splicing effects by RT-PCR. RNAi-knockdown [top panel; control (ctr) versus L knockdown (ΔL)]) and CA-sponge effects [bottom panel; transfection of control (ctr) versus CA-sponge circRNA (CA)] are directly compared, monitoring exon inclusion versus skipping for five predicted target exons, where hnRNP L acts as a repressor (gene names in the middle; β-actin, BA, as an unaffected control). *M*, markers.

### EXAMPLES

### Materials and methods

### In vitro transcription and circularization of short designer circRNAs (Figures 1A, 1B)

RNAs were synthesized by *in vitro* transcription, using double-stranded DNA oligonucleotide templates and the HiScribe™ T7 High Yield RNA Synthesis Kit (NEB), labeling internally by incorporation of azide-modified UTP analog (Jena Bioscience), followed by RQ1 DNase treatment and Sephadex column purification (Sigma-Aldrich). For circularization, T4 RNA ligase (Thermo Fisher Scientific) was used, followed by biotin labeling with copper-free Click Chemistry (DABCO-containing biotin; Jena Bioscience). The sequences of the linear and circular constructs used are set forth below (the underlined regions are complementary allowing the formation of a 6 bp stem to facilitate *in vitro* circularization):
(CA)₁₀, 40 nts
   5'-GGGAGUAAGC (CA)₁₀ GCUUACAGUA-3' (SEQ ID NO:5)
(CA)₁₅, 50 nts
   5'-GGGAGUAAGC (CA)₁₅ GCUUACAGUA-3'(SEQ ID NO:6)
(CA)₂₀, 60 nts
   5'-GGGAGUAAGC (CA)₂₀ GCUUACAGUA-3'(SEQ ID NO:7)
(CA)-SELEX #51, 40 nts
   5'-GGGAGUAAGC (AUACAUGACACACACACGCA) GCUUACAGUA-3' (SEQ ID NO:8)
Negative control, 40 nts
   5'-GGGAGUAAGC (non-specific control sequence) GCUUACAGUA-3'(SEQ ID NO:9)

### Biotin pull-down and RNA-immunoprecipitation (RIP) assays of hnRNP sponging (Figures 1B, 1C)

For *in vitro* binding assays, 50 pmol of biotinylated RNA was prebound to 30 µl Dynabeads MyOne Streptavidin C1 beads (Thermo Fisher Scientific), followed by incubation with 20 µl HeLa cell nuclear extract (CILBiotech) and washing off unbound proteins at 300 mM KCl. Bound proteins were released and separated by 10% SDS-PAGE, followed by Western blotting with hnRNP L- or GAPDH-primary antibodies and peroxidase-coupled secondary antibodies (Sigma-Aldrich).

For assaying hnRNP L sponging *in vivo,* cells were lysed in RIPA buffer (150 mM NaCl, 5 mM EDTA, 0.1% SDS, 1% NP-40, 50 mM Tris pH 7.4), followed by the addition of antibodies (anti-hnRNPL, anti-Flag from Sigma-Aldrich; anti-IMP3 from Millipore) to the pre-cleared lysate, incubation overnight at 4°C, addition to Protein A or Protein G Dynabeads (Thermo Fisher Scientific), and rotation for 2 hr at 4°C. Protein-RNA complexes were washed by increasing the stringency up to 600 mM NaCl. RNA from input and immunoprecipitated fractions was extracted by TRIzol (Ambion), followed by reverse transcription (qScript cDNA Synthesis Kit, Quanta) and PCR with gene-specific primers.

### Stable cell lines (Figure 1C)

HEK293 cell lines that stably express circRNAs were generated, based on a circRNA expression construct [pcDNA5/FRT/TO vector (Thermo Fisher Scientific), using inverted repeats as described by Liang and Wilusz, 2014, Genes Dev 28(20):2233-2247 and with the BGH polyadenylation signal deleted]; genomic integration relied on the tetracycline-inducible Flp-In™ T-Rex™ System (Thermo Fisher Scientific).

*In vitro* transcription and PIE-mediated circularization of long circRNAs (Figures 2A, 2B) RNA was synthesized using the HiScribe™ T7 High Yield RNA Synthesis Kit (New England Biolabs), followed by RQ1 DNase treatment and purification by the Monarch RNA Cleanup Kit (New England Biolabs): (CA)₁₀₀ RNA contains approximately 100 continuous CA-dinucleotides, the negative control RNA of similar length is derived from vector sequences (Rossbach et al., 2009, Mol Cell Biol 29(6): 1442-1451). The (CA)₁₀₀ circular molecule has the sequence (with the T/t residues replaced by U/u residues):
AAAATCCGTTGACCTTAAACGGTCGTGTGGGTTCAAGTCCCTCCACCCCCACGCC GGAAACGCAATAGCCGAAAAACAAAAAACAAAAAAAGAGGATC**cacacacacacacac acacacacacacacacacacacacacacacacacacacacacacacagatccacacgcacacacacacacacacacacac acacacacacacacacacacacacacacacacacagatccacacacgacacacacagatccacacacacacacacacaca cacacacacacacacacacacacacacacacacacacacacaca**AGATCCACTAGTAACGGCTCGAGA AAAAACAAAAAACAAAACGGCTATTATGCGTTACCGGCGAGACGCTACGGACTT (SEQ ID NO:3). This CA-repeat sponge circRNA is 395 nts in total, containing the functional CA-repeat sponge sequence of 219 nts (bold, small letters). The flanking sequences are from the vector sequence required for efficient ribozyme-catalyzed circularization by a group I splicing system [permuted-intron-exon (PIE; Wesselhoeft et al., 2018, Nat Commun 9(1):2629) plus some adapter sequence for cloning. The 219 nts sponge sequence contains a total of 103 (CA)-dinucleotides with some non-CA sequences interspersed that were introduced by the cloning strategy. The sequence of the linear (CA)₁₀₀ is as follows and contains the same 219 nts in the circular (CA)100 molecule (with the T/t residues replaced by U/u residues):

GGGAGACCCAAGCTTCCGTCGATTGTCCACTGGTCAACAATAGATGACTTACAAC
TAATCGGAAGGTGCAGAGACTCGACGGAGCTACCCTAACGTCAAGACGAGGGT
AAAGAGAGAGTCCAATTCTCAAAGCCAATAGGCAGTAGCGAAAGCTGCAAGAGA
ATGAAAATCCGTTGACCTTAAACGGTCGTGTGGGTTCAAGTCCCTCCACCCCCAC
GCCGGAAACGCAATAGCCGAAAAACAAAAAACAAAAAAAGAGGATCcacacacacac
acacacacacacacacacacacacacacacacacacacacacacacagatccacacgcacacacacacacacac
acacacacacacacacacacacacacacacacacacagatccacacacgacacacacagatccacacacacacaca
cacacacacacacacacacacacacacacacacacacacacacaAGATCCACTAGTAACGGCTCGA
GAAAAACAAAAAACAAAACGGCTATTATGCGTTACCGGCGAGACGCTACGGAC
TTAAATAATTGAGCCTTAAAGAAGAAATTCTTTAAGTGGATGCTCTCAAACTCAG
GGAAACCTAAATCTAGTTATAGACAAGGCAATCCTGAGCCAAGCCGAAGTAGTA
ATTAGTAAGACCAGTGGACAATCGACGGATAACAGCATATCTAGA (SEQ ID NO:4).

The negative control circRNA molecule has the sequence (with the T/t residues replaced by U/u residues):
AAAATCCGTTGACCTTAAACGGTCGTGTGGGTTCAAGTCCCTCCACCCCCACGCC GGAAACGCAATAGCCGAAAAACAAAAAACAAAAAAAGAGGATCC**tccggaggaagcgg aacacgtagaaagccagtccgcagaaacggtgctgaccccggatgaatgtcagctactgggctatctggacaagggaaaac gcaagcgcaaagagaaagcaggtagcttgcagtgggcttacatggcgatagctagactgggcggttttatggacagcaagcg aaccggaattgccagctggggcgccctctggtaaggttgggaagccctgcaaagtaaactggatggctttcttgccgccaagg atctgatggcgcaggggatcaagatctgatcaagagacaggatgaggatcgtttcgcatgattacgtctccagtgtgct**CTC GAGAAAAAACAAAAAACAAAACGGCTATTATGCGTTACCGGCGAGACGCTACGG ACTT (SEQ ID NO: 10). This negative control circRNA is 499 nts in total and was used as a negative control of similar length. The flanking regions (capital letters) contain sequences required for efficient ribozyme-catalyzed circularization by a group I splicing system plus some adapter sequence for cloning. In between, a pCR2.1-TOPO vector-derived sequence was cloned (BamHI / XhoI; Rossbach et al., 2009, Mol Cell Biol 29(6): 1442-1451). The sequence of the linear negative control RNA molecule is as follows (with the T/t residues replaced by U/u residues):

GGGAGACCCAAGCTTCCGTCGATTGTCCACTGGTCAACAATAGATGACTTACAAC
TAATCGGAAGGTGCAGAGACTCGACGGGAGCTACCCTAACGTCAAGACGAGGGT

AAAGAGAGAGTCCAATTCTCAAAGCCAATAGGCAGTAGCGAAAGCTGCAAGAGA
ATGAAAATCCGTTGACCTTAAACGGTCGTGTGGGTTCAAGTCCCTCCACCCCCAC
GCCGGAAACGCAATAGCCGAAAAACAAAAAACAAAAAAAGAGGATCCtccggagga
agcggaacacgtagaaagccagtccgcagaaacggtgctgaccccggatgaatgtcagctactgggctatctggacaaggg
aaaacgcaagcgcaaagagaaagcaggtagcttgcagtgggcttacatggcgatagctagactgggcggttttatggacagc
aagcgaaccggaattgccagctggggcgccctctggtaaggttgggaagccctgcaaagtaaactggatggctttcttgccgc
caaggatctgatggcgcaggggatcaagatctgatcaagagacaggatgaggatcgtttcgcatgattacgtctccagtgtgct
CTCGAGAAAAACAAAAAACAAAACGGCTATTATGCGTTACCGGCGAGACGCTA
CGGACTTAAATAATTGAGCCTTAAAGAAGAAATTCTTTAAGTGGATGCTCTCAAA
CTCAGGGAAACCTAAATCTAGTTATAGACAAGGCAATCCTGAGCCAAGCCGAAG
TAGTAATTAGTAAGACCAGTGGACAATCGACGGATAACAGCATATCTAGA (SEQ
ID NO:11).

To induce ribozyme-catalyzed circularization by a group I splicing system [permuted-intron-exon (PIE), Wesselhoeft, *ibid*], splicing buffer (T4 RNA ligase buffer from New England Biolabs) and GTP were added (final concentration of 2 mM), followed by incubation for 8 min at 55°C. Circularization efficiency was checked on the 2% E-Gel system (Thermo Fisher Scientific). To purify circRNAs, RNA was treated with RNase R (Lucigen) and HPLC-fractionated.

### RNA transfection, alternative splicing and validation assays (Figure 2C, Figure 3C)

5 x 10⁴ HeLa cells were seeded one day before transfection, and 100 or 500 ng linear or circular RNA were transfected, using Lipofectamine™ MessengerMax™ mRNA transfection reagent (Thermo Fisher Scientific) and harvesting after 24 hrs. RNA was isolated using TRIzol (Ambion) and RNeasy columns (Qiagen), followed by reverse transcription (qScript cDNA Synthesis Kit, Quanta) and alternative splicing assays by PCR with gene-specific primers. For target validations (Figure 3C), RNA was used from HeLa cells transfected with 500 ng (CA)₁₀₀ circRNA, as described above.

### In vivo expression of long circRNAs (Figures 2A and 2D)

Alternatively to circRNA transfection, circRNA was also generated *in vivo* by transient transfection of circRNA expression constructs, based on the TORNADO system (Litke and Jaffrey, 2019, Nat Biotechnol 37(6):667-375). Four copies of a 20-mer CA-rich RNA, described in our SELEX study as high-affinity binding sequence of hnRNP L (Hui et al., 2005, EMBO J 24(11):1988-1998), and separated by four-nucleotide spacers of AUAU, were cloned as a 92-nucleotide CA-rich RNA region into the TORNADO vector, resulting in the CA-SELEX X4 construct. The CA-SELEX X4 circRNA molecule, 149 nts in total, has the sequence: AACCATGCCGAGTGCGGCCGCGGATCC**atacatgacacacacacgca**atat**atacatgacacacacacg ca**atat**atacatgacacacacacgca**atat**atacatgacacacacacgca**AGATGTGGCCGCGGTCGGCGTG GACTGTAG (SEQ ID NO: 12). This molecule contains four repeats of a 20-mer CA-rich RNA (underlined, bold) and separated by four-nucleotide spacers of AUAU. These repeats were cloned into the TORNADO vector pAV-U6+27-Broccoli (Not I / SacII), by replacing the Broccoli sequence. The flanking regions (capital letters) contain sequences required for efficient ribozyme-catalyzed circularization. After transfection into HeLa cells, alternative splicing changes were monitored by RT-PCR over four days, as described above.

### Global analysis of alternative splicing by RNA-seq, bioinformatic target exon prediction (Figure 3)

For CA-sponge assays, 2.5 x 10⁵ HeLa cells were seeded one day before transfection with 0.5, 1 or 2.5 µg (CA)₁₀₀ circRNA (for comparison, with linear RNA), using Lipofectamine™ MessengerMax™ mRNA transfection rerossbachagent (Thermo Fisher Scientific), and harvesting after 24 hrs. For RNAi-knockdown, 8.8 x 10⁵ cells were reverse-transfected with RNAiMAX (Thermo Fisher Scientific), using siRNAs specific for human hnRNP L and luciferase GL2 (Sigma-Aldrich) (Rossbach et al., 2009, Mol Cell Biol 29(6): 1442-1451). Three days post-transfection, total RNA was isolated, quality-controlled, and depleted of ribosomal RNA (Ribo-Zero Gold rRNA Removal Kit, Epicenter), followed by library preparation (NEBNext Ultra Directional RNA Library Prep Kit) and sequencing on Illumina NextSeq 500 (single-read, 150 bp).

Sequence reads were aligned to the human genome sequence (hg19 assembly) using STAR, an ultrafast universal RNA-seq aligner (Dobin et al., 2013, Bioinformatics 29(1): 15-21). Comprehensive gene annotation from GENCODE Version 19 (gencodegenes.org) was used for gene annotation. The ratio of exon inclusion to skipping junction counts was applied for alternative splicing analysis as described (Rösel et al., 2011, EMBO J 30(10): 1965-1976). Single-exon skipping/inclusion targets upon hnRNP L sponging were predicted, based on dose-dependent increases [0.5, 1, 2.5 µg (CA)₁₀₀ circRNA] of the CA-sponge-dependent effects. For the knockdown samples, target prediction relied on the ratio change between hnRNP L and control knockdown.

### Results

### 1. HnRNP L sponge circRNAs: design, synthesis, specific hnRNP L binding (Figure 1)

To produce circRNAs with specific sponge function for the RNA-binding protein hnRNP L, short RNAs were initially designed that can be efficiently synthesized by *in vitro* T7 transcription and RNA-ligase-mediated circularization (Figure 1A). Based on the known binding specificity of hnRNP L for CA-repeat and CA-rich RNA sequences, we first generated short circRNAs with a common backbone (22-nt stem-loop) and a loop comprised of 10, 15, and 20 CA-dinucleotides, resulting in circRNAs of 40, 50, and 60 nts in total, respectively: (CA)₁₀, (CA)₁₅, and (CA)₂₀. As an alternative option, (CA)-SELEX#51 was used, a 20-nts CA-rich sequence validated as a high-affinity target sequence of hnRNP L (5'-AUACAUGACACACACACGCA-3' (SEQ ID NO: 13); K_{D} 7.2 nM; Hui et al., 2005, EMBO J 24(11):1988-1998). A linear synthetic (CA)₃₂ RNA, which also binds hnRNP L with high affinity (Hui et al., 2003, Nat Struct Biol 10(1):33-37, served as a positive control, a random sequence of 20 nucleotides as a negative control.

To test for hnRNP L sponging *in vitro,* the four short RNAs with 10, 15, 20 CA-dinucleotides or the SELEX#51 hnRNP L high-affinity binding sequence were generated with biotin incorporation, each in linear and circular configuration, as well as the negative control and the linear (CA)₃₂ positive control RNAs. After incubation in nuclear extract from HeLa cells, hnRNP L binding was assayed by pulldown with streptavidin agarose, followed by Western blot analysis for bound hnRNP L, comparing supernatant (2.5%), bound material (20%) and input (2.5%); GAPDH served as input and negative control (Figure 1B). From these *in vitro* assays, it can be concluded that with each of the four high-affinity binding sequences, both in circular and linear form, as well as with the linear RNA molecule of 32 CA dinucleotides "(CA)₃₂ RNA", hnRNP L can be quantitatively bound, in contrast to the negative control RNA.

To assay hnRNP L sponging *in vivo,* we generated HEK293 cell lines, based on the Flp-In™ T-Rex™ system, that stably express - after tetracycline induction - a long circRNA, comprised of approximately 100 CA-dinucleotides, (CA)₁₀₀, as well as a negative control circRNA of similar length (Figure 1C). Lysates were prepared, followed by immunoprecipitation with anti-hnRNP L, or as specificity controls, with anti-IMP3 or anti-FLAG (mock) antibodies.

Immunoprecipitated RNAs were detected by RT-PCR, with primer pairs specific for the designer circRNAs. The (CA)₁₀₀ circRNA binds hnRNP L *in vivo* with high specificity and efficiency. In contrast, the IMP3 protein, another multidomain RNA-binding protein, which recognizes also certain CA-rich sequences (Schneider et al., 2019, Nat Commun 10(1):2266), binds only at a comparatively very low efficiency.

### 2. Designer hnRNP L-sponge circRNA can modulate alternative splicing (Figures 2 and 3)

To demonstrate the functionality of a designer circRNA as hnRNP L sponges, we focused next on the established role of hnRNP L as a specific splicing regulator. We used the (CA)₁₀₀ circRNA (395 nucleotides in total), as well as a negative control circRNA (499 nucleotides), which were synthesized in both linear and circular configuration. For circRNA production we made use of the PIE (permutated exon-intron) system, developed as a very efficient *in vitro* expression platform for long circRNAs, relying on *in vitro* T7 transcription and circular processing through ribozyme-mediated group I splicing (Wesselhoeft et al., 2018, Nat Commun 9(1):2629; Figure 2A). The linear version was produced by T7 transcription from the same construct, but omitting the circular processing step. Both circRNAs were further enriched by RNase R treatment (which digests linear RNAs in the reaction), and both circRNAs and linear RNA transcripts were finally purified by HPLC (Figure 2B).

(CA)₁₀₀ hnRNP L-sponge or control RNAs, either in linear or circular configuration (lin/circ), were transfected in HeLa cells (100 or 500 ng per transfection; Figure 2C). After 24 hrs, alternative splicing was assayed by RT-PCR for two known hnRNP L targets, TJP1 and FALZ, where hnRNP L functions as a repressor during skipping or inclusion of an alternatively spliced exon (Hung et al., 2008, RNA 14(2):284-296). The two RT-PCR products indicate exon inclusion (arrows) and skipping, respectively, and quantitation of exon inclusion (in %) is indicated in the respective lanes.

The CA-repeat circRNA reproducibly and strongly increased exon inclusion, up to 55% (TJP1) and 66% (FALZ). These effects were clearly specific for the CA-repeat sponge RNA (see control transfections), dose-dependent (compare 100 and 500 ng RNA), and much more pronounced for the designer circRNA than for corresponding quantities of linear RNA. The extent of alternative splicing modulation observed here after CA-sponge circRNA transfection was at least comparable with the effects initially found after siRNA-mediated knockdown of hnRNP L expression (Hung et al., 2008, RNA 14(2):284-296). In sum, this indicates that sponging hnRNP L by a CA-repeat circRNA functionally inactivates hnRNP L, resulting in specific alternative splicing changes (for a schematic representation of this new interference principle, see below and Figure 3A).

Alternatively to CA-repeat sequence, a CA-rich sequence for hnRNP L sponging activity was also tested, using a circRNA, which contains CA-rich sequence elements and was expressed *in vivo* in HeLa cells. Specifically, a 20-mer CA-rich RNA sequence was used, which had been characterized as an hnRNP L-high-affinity binding sequence (Hui et al., 2005, EMBO J 24(11): 1988-1998); this sequence also was used in circular form for *in vitro* binding assays, as described above (see Figure 1A and B). For use as an hnRNP L sponge *in vivo,* we assembled four copies of this 20-mer CA-rich RNA sequence, each separated by four-nucleotide spacers of AUAU, resulting in a 92-nucleotide CA-rich RNA region. After cloning in the TORNADO circRNA expression system (Litke and Jaffrey, 2019, Nat Biotechnol 37(6):667-675), a circRNA containing this sequence was transiently expressed in HeLa cells, and induced alternative splicing was monitored over a time course of four days (Figure 2D), based on RT-PCR assays of TJP1 and FALZ, two known alternatively spliced hnRNP L target genes. As demonstrated in the figure, circRNAs with either CA-repeat or CA-rich sequences can efficiently modulate alternative splicing patterns.

### 3. Global analysis of alternative splicing patterns modulated by (CA)₁₀₀ hnRNP L-sponge circRNA (Figure 3)

The foregoing alternative splicing analysis was extended to a genome wide level, focusing on exon skipping and inclusion, the most abundant type of alternative splicing (Figure 3A). It was expected based on the *in vitro* data that the splicing pattern of genes could be altered by the hnRNP L-sponging activity of the (CA)₁₀₀ circRNA. Since hnRNP L can act either as an activator or repressor, the ratio of exon inclusion versus skipping may shift accordingly.

RNA-seq analysis of HeLa cells was used, one-day post-transfection with the (CA)₁₀₀ sponge circRNA, in parallel with a control circRNA transfection (for a schematic flowchart see Figure 3B). In addition, classical siRNA-based hnRNP L knockdown assays were performed to compare RNAi-knockdown of hnRNP L and circRNA-mediated sponging of hnRNP L. Bioinformatics analysis of alternative splicing patterns (and their dose-dependent changes after transfection with different quantities of circRNA) allowed the prediction of target exons that respond in their inclusion or skipping pattern to the CA-sponge circRNA: 109 exons were identified that were repressed by the CA-sponge, of which 44 (40%) responded in the same manner to RNAi knockdown; on the other hand, 102 exons were activated, of which 60 (59%) were RNAi-knockdown-responsive. Validations by semi-quantitative RT-PCR assays of a subset of these predicted targets clearly confirmed our bioinformatics strategy and analysis (Figure 3C). Finally, the high correlation of circRNA-sponge and RNAi-induced effects on exon-specific splicing modulation strongly indicated that circRNA-based sponging results in functional hnRNP L inactivation.

The present invention is particularly directed to the following items:
1. A circular RNA comprising a binding domain having a nucleotide sequence to which one or more copies of an RNA-binding protein binds, thereby capturing and inhibiting the activity of the RNA-binding protein.
2. The circular RNA according to item 1, wherein the nucleotide sequence of the binding domain comprises CA or AC dinucleotides.
3. The circular RNA according to item 2, wherein the RNA-binding protein is heterogeneous nuclear ribonucleoprotein L.
4. The circular RNA according to item 2 or 3, wherein the number of CA or AC dinucleotides is at least 20, at least 30, at least 40, at least 50, at least 60, at least 75, at least 100, at least 125, at least 150, at least 175, at least 200, at least 250, at least 300, at least 350, at least 400, or at least 450 CA or AC dinucleotides.
5. The circular RNA according to any one of items 2 to 4, wherein the number of CA or AC dinucleotides is at least 90 CA or AC dinucleotides.
6. The circular RNA according to any one of items 2 to 5, wherein at least 4 of the CA or AC dinucleotides are consecutive.
7. The circular RNA according to any one of items 2 to 6, wherein the nucleotide sequence of the circular RNA is less than 1000 nucleotides, less than 500 nucleotides, or less than 250 nucleotides in length.
8. The circular RNA according to any one of items 2 to 7, wherein the circular RNA comprises the nucleotide sequence: 
cacacacacacacacacacacacacacacacacacacacacacacacacacacacacacacacacacacacacacacacacacagaucca
cacgcacacacacacacacacacacacacacacacacacacacacacacacacacacacacacacacacacacacacacacacagaucca
cacacgacacacacagauccacacacacacacacacacacacacacacacacacacacacacacacacacacacacacacacacacacac
acacacacacacacaca (SEQ ID NO:14).
9. The circular RNA according to any one of items 1 to 8, wherein the circular RNA comprises a modified ribonucleotide.
10. The circular RNA according to item 9, wherein the modified ribonucleotide is pseudouridine.
11. The circular RNA according to any one of items 1 to 10, wherein the circular RNA comprises a modified backbone.
12. The circular RNA according to item 11, wherein the modified backbone comprises a phosphorothioate internucleotide linkage.
13. The circular RNA according to any one of items 1 to 12, wherein the circular RNA is purified.
14. A pharmaceutical composition comprising the circular RNA according to any one of items 1 to 13, and a pharmaceutically acceptable carrier.
15. A method of treating or preventing a disease or disorder characterized by an increased amount or increased activity of an RNA-binding protein, comprising administering to a patient in need thereof the circular RNA according to any one of items 1 to 13 or the pharmaceutical composition according to item 14.
16. The method according to item 15, wherein the disease or disorder is cancer.
17. The method according to item 16, wherein the cancer is characterized by one or more solid tumors.
18. The method according to item 16 or 17, wherein the cancer is pancreatic cancer or oral squamous cell carcinoma.
19. The method according to any one of items 16 to 18, wherein the circular RNA is administered intravenously or intraarterially or is administered by injection into the solid tumor.
20. A pharmaceutical composition according to item 14 for use as a medicament.
21. A pharmaceutical composition according to item 14 for use in treating or preventing a disease or disorder characterized by an increased amount or increased activity of an RNA-binding protein.
23. The pharmaceutical composition for the use according to item 21, wherein the disease or disorder is cancer.
24. The pharmaceutical composition for the use according to item 23, wherein the cancer is characterized by one or more solid tumors.
25. The pharmaceutical composition for the use according to item 23 or 24, wherein the cancer is pancreatic cancer or oral squamous cell carcinoma.
26. The pharmaceutical composition for the use according to any one of items 23 to 25, wherein the circular RNA is administered intravenously or intraarterially or is administered by injection into the solid tumor.
27. A method for capturing an RNA-binding protein comprising contacting the RNA-binding protein with a circular RNA, wherein the circular RNA comprises a binding domain having a nucleotide sequence to which one or more copies of the RNA-binding protein binds.
28. The method according to item 27, wherein binding of the RNA-binding protein to the circular RNA inhibits the metabolic activity of the RNA-binding protein.
29. A method of inhibiting the activity of an RNA-binding protein comprising contacting the RNA-binding protein with a circular RNA, wherein the circular RNA comprises a binding domain having a nucleotide sequence to which one or more copies of the RNA-binding protein binds thereby inhibiting the metabolic activity of the RNA-binding protein.
30. A method of forming a complex comprising a circular RNA and an RNA-binding protein, comprising contacting the circular RNA with an RNA-binding protein, wherein the circular RNA comprises a binding domain having a nucleotide sequence to which one or more copies of the RNA-binding protein binds.
31. The method according to item 30, wherein the RNA-binding protein in the complex is unable to perform its metabolic activity.
32. The method according to any one of items 27 to 31, wherein contacting takes place *in vitro.*
33. The method according to any one of items 27 to 31, wherein contacting takes place *in vivo.*

## Claims

1. A circular RNA comprising a binding domain having a nucleotide sequence to which one or more copies of an RNA-binding protein binds, thereby capturing and inhibiting the activity of the RNA-binding protein.

2. The circular RNA according to claim 1, wherein the nucleotide sequence of the binding domain comprises CA or AC dinucleotides.

3. The circular RNA according to claim 2, wherein the RNA-binding protein is heterogeneous nuclear ribonucleoprotein L.

4. The circular RNA according to claim 2 or 3, wherein the number of CA or AC dinucleotides is at least 20, at least 30, at least 40, at least 50, at least 60, at least 75, at least 100, at least 125, at least 150, at least 175, at least 200, at least 250, at least 300, at least 350, at least 400, or at least 450 CA or AC dinucleotides.

5. The circular RNA according to any one of claims 2 to 4, wherein the number of CA or AC dinucleotides is at least 90 CA or AC dinucleotides.

6. The circular RNA according to any one of claims 2 to 5, wherein at least 4 of the CA or AC dinucleotides are consecutive.

7. The circular RNA according to any one of claims 2 to 6, wherein the nucleotide sequence of the circular RNA is less than 1000 nucleotides, less than 500 nucleotides, or less than 250 nucleotides in length.

8. The circular RNA according to any one of claims 2 to 7, wherein the circular RNA comprises the nucleotide sequence:

cacacacacacacacacacacacacacacacacacacacacacacacacacacacacacacacacagaucca

cacgcacacacacacacacacacacacacacacacacacacacacacacacacacacacacacagaucca

cacacgacacacacagauccacacacac acacacacacacacacacacacacacacacacacacacacac

acacacacacacaca (SEQ ID NO:14).

9. A pharmaceutical composition comprising the circular RNA according to any one of claims 1 to 8, and a pharmaceutically acceptable carrier.

10. A method of treating or preventing a disease or disorder **characterized by** an increased amount or increased activity of an RNA-binding protein, comprising administering to a patient in need thereof the circular RNA according to any one of claims 1 to 8 or the pharmaceutical composition according to claim 9.

11. The method according to claim 10, wherein the disease or disorder is cancer.

12. The method according to claim 11, wherein the cancer is pancreatic cancer or oral squamous cell carcinoma.

13. A method for capturing an RNA-binding protein comprising contacting the RNA-binding protein with a circular RNA, wherein the circular RNA comprises a binding domain having a nucleotide sequence to which one or more copies of the RNA-binding protein binds, optionally wherein binding of the RNA-binding protein to the circular RNA inhibits the metabolic activity of the RNA-binding protein.

14. A method of forming a complex comprising a circular RNA and an RNA-binding protein, comprising contacting the circular RNA with an RNA-binding protein, wherein the circular RNA comprises a binding domain having a nucleotide sequence to which one or more copies of the RNA-binding protein binds, optionally wherein the RNA-binding protein in the complex is unable to perform its metabolic activity.

15. The method according to claim 13 or 14, wherein contacting takes place *in vitro.*


SEQUENCE LISTING

<110> Justus-Liebig-Universität

<120> Circular RNA and Uses Thereof for Inhibiting RNA-Binding Proteins

<130> TM 1070

<160> 14

<170> BiSSAP 1.3.5

<210> 1
<211> 219
<212> DNA
<213> artifical sequence

<223> "219 nucleotides"

<220>
<223> 219 nucleotides

<400> 1
cacacacaca cacacacaca cacacacaca cacacacaca cacacacaca cacacacaca 60

cagatccaca cgcacacaca cacacacaca cacacacaca cacacacaca cacacacaca 120

cacacacaca gatccacaca cgacacacac agatccacac acacacacac acacacacac 180

acacacacac acacacacac acacacacac acacacaca 219


<210> 2
<211> 149
<212> DNA
<213> artificial sequence

<223> "149 nucleotides"

<220>
<223> 149 nucleotides

<400> 2
aaccatgccg agtgcggccg cggatccata catgacacac acacgcaata tatacatgac 60

acacacacgc aatatataca tgacacacac acgcaatata tacatgacac acacacgcaa 120

gatgtggccg cggtcggcgt ggactgtag 149


<210> 3
<211> 395
<212> DNA
<213> artificial sequence

<223> "395 nucleotides"

<220>
<223> 395 nucleotides

<400> 3
aaaatccgtt gaccttaaac ggtcgtgtgg gttcaagtcc ctccaccccc acgccggaaa 60

cgcaatagcc gaaaaacaaa aaacaaaaaa agaggatcca cacacacaca cacacacaca     120

cacacacaca cacacacaca cacacacaca cacacacaca gatccacacg cacacacaca     180

cacacacaca cacacacaca cacacacaca cacacacaca cacacacaga tccacacacg     240

acacacacag atccacacac acacacacac acacacacac acacacacac acacacacac     300

acacacacac acacacaaga tccactagta acggctcgag aaaaaacaaa aaacaaaacg     360

gctattatgc gttaccggcg agacgctacg gactt                                395


<210> 4
<211> 713
<212> DNA
<213> artificial sequence

<223> "713 nucleotides"

<220>
<223> 713 nucleotides

<400> 4
gggagaccca agcttccgtc gattgtccac tggtcaacaa tagatgactt acaactaatc      60

ggaaggtgca gagactcgac gggagctacc ctaacgtcaa gacgagggta aagagagagt     120

ccaattctca aagccaatag gcagtagcga aagctgcaag agaatgaaaa tccgttgacc     180

ttaaacggtc gtgtgggttc aagtccctcc acccccacgc cggaaacgca atagccgaaa     240

aacaaaaaac aaaaaaagag gatccacaca cacacacaca cacacacaca cacacacaca     300

cacacacaca cacacacaca cacacagatc cacacgcaca cacacacaca cacacacaca     360

cacacacaca cacacacaca cacacacaca cacagatcca cacacgacac acacagatcc     420

acacacacac acacacacac acacacacac acacacacac acacacacac acacacacac     480

acaagatcca ctagtaacgg ctcgagaaaa aacaaaaaac aaaacggcta ttatgcgtta     540

ccggcgagac gctacggact taaataattg agccttaaag aagaaattct ttaagtggat     600

gctctcaaac tcagggaaac ctaaatctag ttatagacaa ggcaatcctg agccaagccg     660

aagtagtaat tagtaagacc agtggacaat cgacggataa cagcatatct aga            713


<210> 5
<211> 40
<212> RNA
<213> artificial sequence

<223> "40 nucleotides"

<220>
<223> 40 nucleotides

<400> 5
gggaguaagc cacacacaca cacacacaca gcuuacagua                            40

<210> 6
<211> 50
<212> RNA
<213> artificial sequence

<223> "50 nucleotides"

<220>
<223> 50 nucleotides

<400> 6
gggaguaagc cacacacaca cacacacaca cacacacaca gcuuacagua                50


<210> 7
<211> 60
<212> RNA
<213> artifical sequence

<223> "60 nucleotides"

<220>
<223> 60 nucleotides

<400> 7
gggaguaagc cacacacaca cacacacaca cacacacaca cacacacaca gcuuacagua      60


<210> 8
<211> 40
<212> RNA
<213> artificial sequence

<223> "40 nucleotides"

<220>
<223> 40 nucleotides

<400> 8
gggaguaagc auacaugaca cacacacgca gcuuacagua                           40


<210> 9
<211> 40
<212> RNA
<213> artificial sequence

<223> "non-specific control sequence n could be a, c, u or g"

<220>
<223> non-specific control sequence n could be a, c, u or g

<220>
<221> misc_feature
<222> 11..30
<223> /note="n is a, c, g, or u"

<400> 9
gggaguaagc nnnnnnnnnn nnnnnnnnnn gcuuacagua                           40


<210> 10
<211> 499

<212> DNA
<213> artificial sequence

<223> "499 nucleotides"

<220>
<223> 499 nucleotides

<400> 10
aaaatccgtt gaccttaaac ggtcgtgtgg gttcaagtcc ctccaccccc acgccggaaa 60

cgcaatagcc gaaaaacaaa aaacaaaaaa agaggatcct ccggaggaag cggaacacgt 120

agaaagccag tccgcagaaa cggtgctgac cccggatgaa tgtcagctac tgggctatct 180

ggacaaggga aaacgcaagc gcaaagagaa agcaggtagc ttgcagtggg cttacatggc 240

gatagctaga ctgggcggtt ttatggacag caagcgaacc ggaattgcca gctggggcgc 300

cctctggtaa ggttgggaag ccctgcaaag taaactggat ggctttcttg ccgccaagga 360

tctgatggcg caggggatca agatctgatc aagagacagg atgaggatcg tttcgcatga 420

ttacgtctcc agtgtgctct cgagaaaaaa caaaaaacaa aacggctatt atgcgttacc 480

ggcgagacgc tacggactt 499

<210> 11
<211> 817
<212> DNA
<213> artificial sequence

<223> "817 nucleotides"

<220>
<223> 817 nucleotides

<400> 11
gggagaccca agcttccgtc gattgtccac tggtcaacaa tagatgactt acaactaatc 60

ggaaggtgca gagactcgac gggagctacc ctaacgtcaa gacgagggta aagagagagt 120

ccaattctca aagccaatag gcagtagcga aagctgcaag agaatgaaaa tccgttgacc 180

ttaaacggtc gtgtgggttc aagtccctcc acccccacgc cggaaacgca atagccgaaa 240

aacaaaaaac aaaaaaagag gatcctccgg aggaagcgga acacgtagaa agccagtccg 300

cagaaacggt gctgaccccg gatgaatgtc agctactggg ctatctggac aagggaaaac 360

gcaagcgcaa agagaaagca ggtagcttgc agtgggctta catggcgata gctagactgg 420

gcggttttat ggacagcaag cgaaccggaa ttgccagctg gggcgccctc tggtaaggtt 480

gggaagccct gcaaagtaaa ctggatggct ttcttgccgc caaggatctg atggcgcagg 540

ggatcaagat ctgatcaaga gacaggatga ggatcgtttc gcatgattac gtctccagtg 600

tgctctcgag aaaaaacaaa aaacaaaacg gctattatgc gttaccggcg agacgctacg 660

gacttaaata attgagcctt aaagaagaaa ttctttaagt ggatgctctc aaactcaggg 720

aaacctaaat ctagttatag acaaggcaat cctgagccaa gccgaagtag taattagtaa 780

gaccagtgga caatcgacgg ataacagcat atctaga 817

<210> 12
<211> 149
<212> DNA
<213> artificial sequence

<223> "149 nucleotides"

<220>
<223> 149 nucleotides

<400> 12
aaccatgccg agtgcggccg cggatccata catgacacac acacgcaata tatacatgac 60

acacacacgc aatatataca tgacacacac acgcaatata tacatgacac acacacgcaa 120

gatgtggccg cggtcggcgt ggactgtag 149

<210> 13
<211> 20
<212> RNA
<213> artificial sequence

<223> "20 nucleotides"

<220>
<223> 20 nucleotides

<400> 13
auacaugaca cacacacgca 20

<210> 14
<211> 219
<212> RNA
<213> artificial sequence

<223> "219 nucleotides"

<220>
<223> 219 nucleotides

<400> 14
cacacacaca cacacacaca cacacacaca cacacacaca cacacacaca cacacacaca 60

cagauccaca cgcacacaca cacacacaca cacacacaca cacacacaca cacacacaca 120

cacacacaca gauccacaca cgacacacac agauccacac acacacacac acacacacac 180

acacacacac acacacacac acacacacac acacacaca 219